# EUROPEAN PATENT SPECIFICATION

(11) **EP 2 425 715 B1**
(45) Date of publication and mention of the grant of the patent: **05.03.2014**
(21) Application number: 11185780.1
(22) Date of filing: 28.08.2006
(51) Int. Cl.: A01N 37/18, C07C 233/00, A61K 31/165, A61P 13/12, A61P 17/02, A61P 19/00

(54) **Treating symptoms of renal disease with selective androgen receptor modulators (SARM)**
Behandlung von Symptomen von Nierenerkrankungen mithilfe von selektiven Androgenrezeptormodulatoren (SARM)
Traitement de symptomes de troubles rénaux avec des modulateurs sélectifs de récepteurs d'androgènes

(30) Priority: 31.08.2005 US 712390 P
(43) Date of publication of application: 07.03.2012
(62) Divisional of application: 06813845.2
(73) Proprietor: University of Tennessee Research Foundation, Knoxville, TN 37996-1527 (US)
(72) Inventor: Dalton, James T, Upper Arlington, OH 43221 (US); Miller, Duane D., Germantown, TN 38139 (US)
(74) Representative: Pearl Cohen Zedek Latzer Baratz UK LLP

(56) References cited:
- WO-A2-2004/035736
- US-A- 5 591 736
- US-A1- 2005 033 074
- US-A1- 2005 176 692

## Description

### FIELD OF INVENTION

This invention relates to the prevention and treatment of renal disease, burns, wounds, spinal cord injuries and conditions involving involuntary weight loss and/or hypgonadism. More particularly, this invention relates to a method of treating, preventing, suppressing, inhibiting, or reducing the incidence of end-stage renal disease, bums, wounds and/or spinal cord injury in a subject, by administering to the subject a selective androgen receptor modulator (SARM) compound and/or its analog, derivative, isomer, metabolite, pharmaceutically acceptable salt, pharmaceutical product, hydrate, N-oxide, prodrug, polymorph, impurity or crystal, or any combination thereof.

### BACKGROUND OF THE INVENTION

A wide variety of diseases and/or conditions are affected by hypogonadism, and catabolic effects, including kidney disease, central nervous system injuries, burns and chronic wounds.

In the United States (US), there is a rising incidence and prevalence of kidney failure. The number of patients enrolled in end-stage renal disease (ESRD) Medicare-funded programs has increased from approximately 10,000 beneficiaries in 1973 to 86,354 in 1983, and to 431,284 as of December 31,2002. In 2002 alone, 100,359 patients entered the US ESRD program. Chronic kidney disease (CKD) is a precursor to ESRD and occurs when the kidneys are not able to adequately remove wastes from the body. CKD is a slowly progressing disease, in which diabetes, hypertension and anemia may be co-morbid conditions.

CKD is diagnosed using a staging system that demonstrates the amount of kidney function available (stage 1 = normal kidney function) and patients often do not present symptoms in the early stages. Stage 5 of CKD is ESRD, which is a complete or near complete failure of the kidneys and usually occurs when kidney function is less than 10% of baseline.

Accompanying symptoms associated with ESRD include hypogonadism, involuntary weight loss, fatigue and others.

Burns result in a testosterone reduction, nitrogen level reduction and a reduction in bone mineral density (BMD), which may persist even as long one year following the injury and is associated with impaired wound healing, increased infection risks, erosion of lean body mass, hampered rehabilitation, and delayed reintegration of burn survivors into society. Catabolic effects initiated as a result of the hum lead to significant involuntary weight loss, further compounding the problem.

Spinal cord injuries may result in the alteration central neurotransmitter secretion or production, which in turn may cause a hypothalamus-pituitary-adrenal axis dysfunction, leading to decreases in testosterone and other hormone levels. SCI or other acute illness or trauma characteristically includes heightened catabolism in conjunction with the lowered anabolic activity resulting in a condition that is prone to loss of lean body tissue. As long as the catabolic process goes uninterrupted, disturbed nutrient utilization will continue. The effects of the loss of lean body mass include the development of wounds and impaired healing mechanisms. Because of poor nutrition and protein combined with immobilization, patients with spinal cord injury are at high risk for bed sores.

Chronic wounds may be caused by any number of conditions, including diabetes, circulatory problems, immobilization and others. Compounding the problem, for example in diabetes, is the presence of neuropathy, which increases the risk of foot ulceration.

While there are many treatments and therapies for these conditions, none are ideal. Since the androgen receptor (AR) signaling pathway has been shown to increase lean muscle mass, muscle strength and muscle protein synthesis, and since hypogonadism accompanies these conditions, molecules targeting the AR signaling pathway may be useful in treating these diseases and/or conditions.

### SUMMARY OF THE INVENTION

This invention provides: 1) a method of treating a subject suffering from, or predisposed to a kidney disease or disorder; 2) a method of treating a subject suffering from a wound, or reducing the incidence of, or mitigating the severity of, or enhancing or hastening healing of a wound in a subject; 3) a method of treating a subject suffering from a burn, or reducing the incidence of, or mitigating the severity of a burn in a subject; 4) a method of treating a subject suffering from a spinal cord injury, comprising the step of administering to said subject a selective androgen receptor modulator (SARM) and/or an analog, derivative, isomer, metabolite, pharmaceutically acceptable salt, pharmaceutical product, hydrate, *N*-oxide, prodrug, polymorph, impurity or crystal of said SARM compound, or any combination thereof.

In one embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound of formula I and/or its analog, derivative, isomer, metabolite, pharmaceutically acceptable salt, pharmaceutical product, hydrate, *N*-oxide, impurity, prodrug, polymorph, crystal, or any combination thereof: 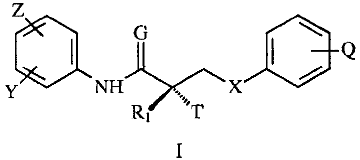
wherein G is O or S;
X is a bond, O, CH₂, NH, Se, PR, NO or NR;
T is OH, OR, NHCOCH₃, or NHCOR
Z is NO₂, CN, COOH, COR, NHCOR or CONHR;
Y is CF₃, F, I, Br, Cl, CN, C(R)₃ or Sn(R)₃;
Q is alkyl, halogen, CF₃, CN, C(R)₃, Sn(R)₃, N(R)₂, NHCOCH₃, NHCOCF₃, NHCOR, NHCONHR, NHCOOR, OCONHR, CONHR, NHCSCH₃, NHCSCF₃,NHCSR NHSO₂CH₃, NHSO₂R, OR, COR, OCOR, OSO₂R, SO₂R, SR; or Q together with the benzene ring to which it is attached is a fused ring system represented by structure A, B or C: 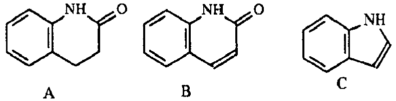
   R is alkyl, haloalkyl, dihaloalkyl, trihaloalkyl, CH₂F, CHF₂, CF₃,CF₂CF₃, aryl, phenyl, halogen, alkenyl or OH; and
   R₁ is CH₃, CH₂F, CHF₂, CF₃, CH₂CH₃, or CF₂CF₃.

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound of formula II and/or its analog, derivative, isomer, metabolite, pharmaceutically acceptable salt, pharmaceutical product, hydrate, *N*-oxide, impurity, prodrug, polymorph, crystal, or any combination thereof: 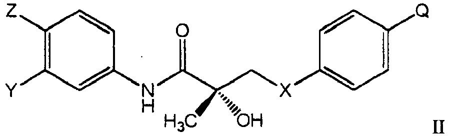
wherein X is a bond, O, CH₂, NH, Se, PR, NO or NR;
Z is NO₂, CN, COOH, COR, NHCOR or CONHR;
Y is CF₃, F, I, Br, Cl, CN, C(R)₃ or Sn(R)₃;
Q is alkyl, halogen, CF₃, CN, C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OR, COR, OCOR, OSO₂R, SO₂R, SR; or Q together with the benzene ring to which it is attached is a fused ring system represented by structure A, B or C: 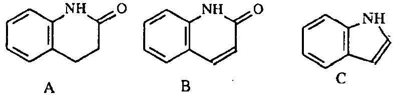 R is alkyl, haloalkyl, dihaloalkyl, trihaloalkyl, CH₂F, CHF₂, CF₃, CF₂CF₃, aryl, phenyl, halogen, alkenyl or OH.

In one embodiment, the SARM compound is a compound of formula II wherein X is O. In another embodiment, the SARM compound is a compound of formula II wherein Y is CF₃. In another embodiment, the SARM compound is a compound of formula II wherein Z is NO₂. In another embodiment, the SARM compound is a compound of formula II wherein Z is CN. In another embodiment, the SARM compound is a compound of formula II wherein Q is halogen. i.e. F, Cl, Br or I. In another embodiment, the SARM compound is a compound of formula II wherein Q is NHCOCH₃.

In another embodiment, the SARM compound is a compound of formula II wherein X is O, Z is NO₂, Y is CF₃ and Q is halogen. In another embodiment, the SARM compound is a compound of formula II wherein X is O, Z is CN, Y is CF₃ and Q is halogen. In another embodiment, the SARM compound is a compound of formula II wherein X is O, Z is CN, Y is CF₃ and Q is CN.

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury,, is a compound of formula III and/or its analog, derivative, isomer, metabolite, pharmaceutically acceptable salt, pharmaceutical product, hydrate, *N*-oxide, impurity, prodrug, polymorph, crystal, or any combination thereof: 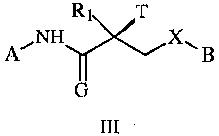
wherein X is a bond, O, CH₂, NH, Se, PR, NO or NR;
G is O or S ;
R₁ is CH₃, CH₂F, CHF₂, CF₃, CH₂CH₃ or CF₂CF₃;
T is OH, OR, NHCOCH₃, or NHCOR;
R is alkyl, haloalkyl, dihaloalkyl, trihaloalkyl, CH₂F, CHF₂, CF₃, CF₂CF₃, aryl, phenyl, halogen, alkenyl or OH;
A is a ring selected from: 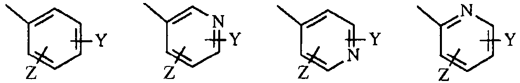 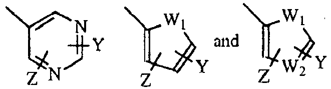
B is a ring selected from: 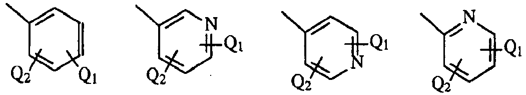 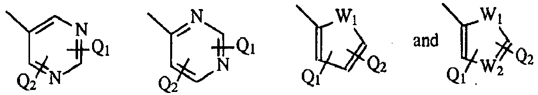
   wherein A and B cannot simultaneously be a benzene ring;
   Z is NO₂, CN, COOH, COR, NHCOR or CONHR;
   Y is CF₃, F, I, Br, Cl, CN, C(R)₃ or Sn(R)₃;
   Q₁ and Q₂ are independently of each other a hydrogen, alkyl, halogen, CF₃, CN, C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OR, COR, OCOR, OSO₂R, SO₂R, SR, 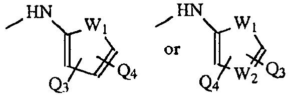
      Q₃ and Q₄ are independently of each other a hydrogen, alkyl, halogen, CF₃, CN, C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR NHSO₂CH_{3,} NHSO₂R, OR, COR, OCOR, OSO₂R, SO₂R or SR;
      W₁ is O, NH, NR, NO or S; and
      W₂ is N or NO.

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound of formula IV and/or its analog, derivative, isomer, metabolite, pharmaceutically acceptable salt, pharmaceutical product, hydrate, *N*-oxide, impurity, prodrug, polymorph, crystal, or any combination thereof: 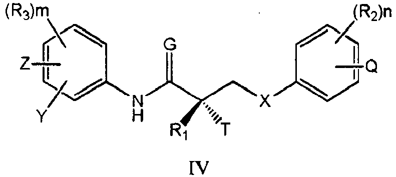
wherein X is a bond, O, CH₂, NH, Se, PR, NO or NR;
G is O or S ;
T is OH, OR, -NHCOCH₃, or NHCOR;
R is alkyl, haloalkyl, dihaloalkyl, trihaloalkyl, CH₂F, CHF₂, CF₃, CF₂CF₃, aryl, phenyl, halogen, alkenyl or OH;
R₁ is CH₃, CH₂F, CHF₂, CF₃, CH₂CH₃, or CF₂CF₃;
R₂ is F, Cl, Br, I, CH₃, CF₃, OH, CN, NO₂, NHCOCH₃, NHCOCF₃, NHCOR, alkyl, arylalkyl, OR, NH₂, NHR, N(R)₂, SR;
R₃ is F, Cl, Br, I, CN, NO₂, COR, COOH, CONHR, CF₃, Sn(R)₃, or (R)₃ together with the benzene ring to which it is attached forms a fused ring system represented by the structure: 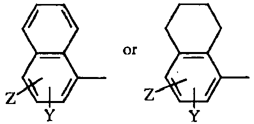
   Z is NO₂, CN, COR, COOH, or CONHR;
   Y is CF₃, F, Br, Cl, I, CN, or Sn(R)₃;
   Q is H, alkyl, halogen, CF₃, CN, C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OH, OR, COR, OCOR, OSO₂R SO₂R, SR; or Q together with the benzene ring to which it is attached is a fused ring system represented by structure A, B or C: 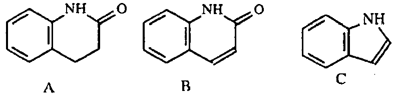
      n is an integer of 1-4; and
      m is an integer of 1-3.

In one embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound represented by the structure of formula VI and/or its analog, derivative, isomer, metabolite, pharmaceutically acceptable salt, pharmaceutical product, hydrate, *N*-oxide, impurity, prodrug, polymorph, crystal, or any combination thereof:

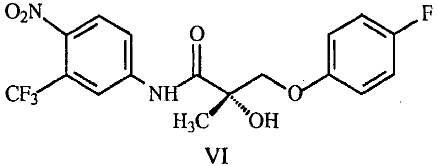

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound represented by the structure of formula VII and/or its analog, derivative, isomer, metabolite, pharmaceutically acceptable salt, pharmaceutical product, hydrate, *N*-oxide, impurity, prodrug, polymorph, crystal, or any combination thereof:

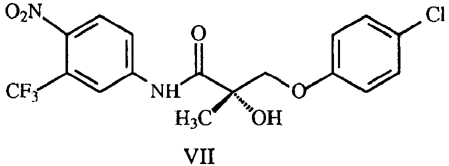

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound represented by the structure of formula VIII and/or its analog, derivative, isomer, metabolite, pharmaceutically acceptable salt, pharmaceutical product, hydrate, *N*-oxide, impurity, prodrug, polymorph, crystal, or any combination thereof:

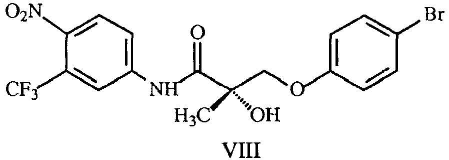

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a. subject suffering from a burn, or reducing the incidence of, or mitigating the severity of a burn in a subject; and/or 4) treating a subject suffering from a spinal cord injury, is a compound represented by the structure of formula IX and/or its analog, derivative, isomer, metabolite, pharmaceutically acceptable salt, pharmaceutical product, hydrate, *N*-oxide, impurity, prodrug, polymorph, crystal, or any combination thereof:

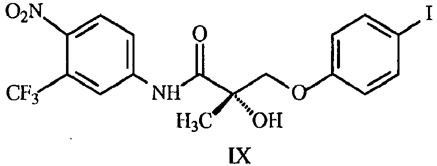

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound represented by the structure of formula X and/or its analog, derivative, isomer, metabolite, pharmaceutically acceptable salt, pharmaceutical product, hydrate, *N*-oxide, impurity, prodrug, polymorph, crystal, or any combination thereof:

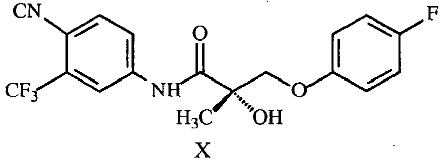

In another embodiment, the SARM compound which is effective at 1) treatingasubject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound represented by the structure of formula XI and/or its analog, derivative, isomer, metabolite, pharmaceutically acceptable salt, pharmaceutical product, hydrate, *N*-oxide, impurity, prodrug, polymorph, crystal, or any combination thereof:

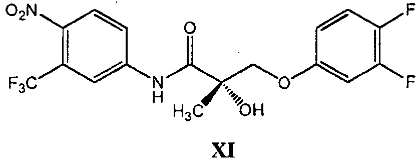

In another embodiment, the SARM compound which is effective at 11) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound represented by the structure of formula XII and/or its analog, derivative, isomer, metabolite, pharmaceutically acceptable salt, pharmaceutical product, hydrate, *N*-oxide, impurity, prodrug, polymorph, crystal, or any combination thereof:

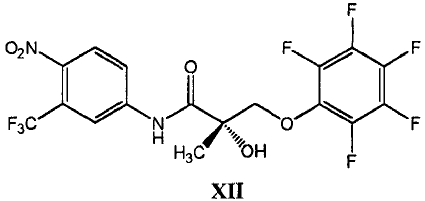

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound represented by the structure of formula XIII and/or its analog, derivative, isomer, metabolite, pharmaceutically acceptable salt, pharmaceutical product, hydrate, *N*-oxide, impurity, prodrug, polymorph, crystal, or any combination thereof: 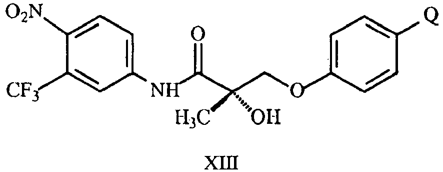 wherein Q is as defined above for formula I.

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound represented by the structure of formula XIV and/or its analog, derivative, isomer, metabolite, pharmaceutically acceptable salt, pharmaceutical product, hydrate, *N*-oxide, impurity, prodrug, polymorph, crystal, or any combination thereof: 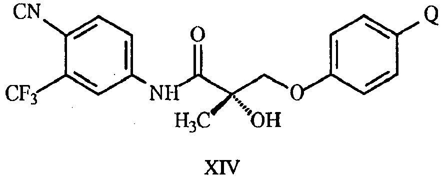 wherein Q is as defined above for formula I.

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn; or reducing the incidence of, or mitigating the severity of a burn in a subject; and/or 4) treating a subject suffering from a spinal cord injury, is a compound represented by the structure of formula XVI and/or its analog, derivative, isomer, metabolite, pharmaceutically acceptable salt, pharmaceutical product, hydrate, *N*-oxide, impurity, prodrug, polymorph, crystal, or any combination thereof:

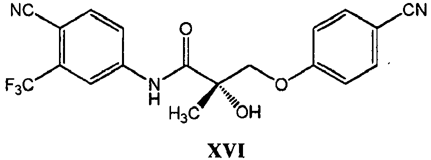

In another embodiment, the SARM compound which is effective at 1) treating a subject suffering from, or predisposed to a kidney disease or disorder; 2) treating a subject suffering from a wound, or reducing the incidence of, or mitigating the severity of a wound in a subject; 3) treating a subject suffering from a burn, or reducing the incidence of, or mitigating the severity of a burn in a subject; and/or 4) treating a subject suffering from a spinal cord injury, is a compound represented by the structure of formula XVII, XVIII, XIX, XX, and/or its analog, derivative, isomer, metabolite, pharmaceutically acceptable salt, pharmaceutical product, hydrate, *N-*oxide, impurity, prodrug, polymorph, crystal, or any combination thereof:

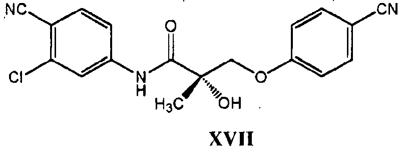

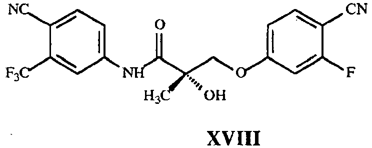

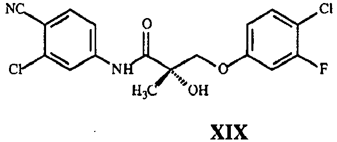

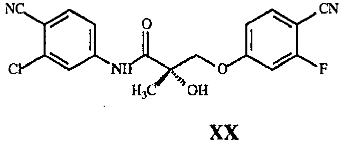

In one embodiment, the administration comprises administering a pharmaceutical composition comprising the SARM, and a pharmaceutically acceptable carrier.

### DETAILED DESCRIPTION OF THE INVENTION

This invention provides: 1) a method of treating a subject suffering from, or predisposed to a kidney disease or disorder; 2) a method of treating a subject suffering from a wound, or reducing the incidence of, or mitigating the severity of a wound in a subject; 3) a method of treating a subject suffering from a burn, or reducing the incidence of, or mitigating the severity of a burn in a subject; 4) a method of treating a subject suffering from a spinal cord injury, comprising the step of administering to said subject a selective androgen receptor modulator (SARM) and/or an analog, derivative, isomer, metabolite, pharmaceutically acceptable salt, pharmaceutical product, hydrate, *N*-oxide, prodrug, polymorph, impurity or crystal of said SARM compound, or any combination thereof.

### Selective Androgen Receptor Modulators (SARMS)

Selective androgen receptor modulators (SARMs) are a class of androgen receptor targeting agents (ARTA), which demonstrate, in one embodiment, androgenic, or in another embodiment, anabolic activity, or in another embodiment, a combination thereof. In one embodiment, different SARMs, while possessing both androgenic and anabolic activity, may differ in terms of relative activity between the two, such that, for example, some SARMs are more anabolic and less androgenic, or in another embodiment, more androgenic and less anabolic, or in another embodiment, both highly anabolic and androgenic, or in another embodiment, any activity level therebetween. The choice of SARM may reflect a need for any level of such activity, and such choice represents an embodiment of this invention.

In another embodiment, the SARM activity may also reflect the genetic background of the subject to which the SARM is administered. SARMs bind androgen receptors in a subject, and genetic influences upon such binding, in terms of, in one embodiment, the receptor, or in another embodiment, level of endogenous hormone production may impact the SARM activity in the subject. Such considerations, and in one embodiment, tailoring of choice of SARM for a particular subject, representing, in one embodiment, personalized medicine in terms of specific choice of therapeutic used represents another embodiment of this invention.

SARMs are nonsteroidal ligands for the androgen receptor. In one embodiment, these agents are useful for the treatment of a variety of hormone-related conditions such as sexual dysfunction, decreased sexual libido, erectile dysfunction, hypogonadism, sarcopenia, osteopenia, osteoporosis, alterations in cognition and mood, depression, anemia, hair loss, obesity, benign prostate hyperplasia and/or prostate cancer. Further, SARMs are useful for oral testosterone replacement therapy, and imaging prostate cancer. SARMs are useful in the treatment of a variety of diseases, disorders and/or conditions, which are affected by androgen binding to its receptor, and are therefore useful in treating such in males and/or female. For example, SARMs are useful in females for the treatment of a variety of hormone-related conditions including, such as sexual dysfunction, decreased sexual libido, hypogonadism, sarcopenia, osteopenia, osteoporosis, alterations in cognition and mood, depression, anemia, hair loss, obesity, endometriosis, breast cancer, uterine cancer and ovarian cancer.

In one embodiment, the SARM compound which is effective for the methods of this invention, will be characterized by the structure of formula I: 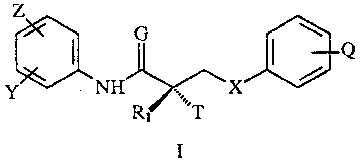
wherein G is O or S;
X is a bond, O, CH₂, NH, Se, PR, NO or NR;
T is OH, OR, NHCOCH₃, or NHCOR
Z is NO₂, CN, COOH, COR, NHCOR or CONHR;
Y is CF₃, F, I, Br, Cl, CN, C(R)₃ or Sn(R)₃;
Q is alkyl, halogen, CF₃, CN, C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OR, COR, OCOR, OSO₂R, SO₂R, SR; or Q together with the benzene ring to which it is attached is a fused ring system represented by structure A, B or C: 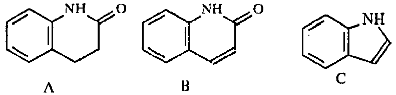
   R is alkyl, haloalkyl, dihaloalkyl, trihaloalkyl, CH₂F, CHF₂ CF₃,CF₂CF₃, aryl, phenyl, halogen, alkenyl or OH; and
   R₁ is CH₃, CH₂F, CHF₂, CF₃, CH₂CH₃, or CF₂CF₃.

In one embodiment, the SARM is an analog of the compound of formula I. In another embodiment, the SARM is a derivative of the compound of formula I. In another embodiment, the SARM is an isomer of the compound of formula I. In another embodiment, the SARM is a metabolite of the compound of formula 1. In another embodiment, the SARM is a pharmaceutically acceptable salt of the compound of formula I. In another embodiment, the SARM is a pharmaceutical product of the compound of formula I. In another embodiment, the SARM is a hydrate of the compound of formula I. In another embodiment, the SARM is an *N*-oxide of the compound of formula 1. In another embodiment, the SARM is a crystal of the compound of formula I. In another embodiment, the SARM is a polymorph of the compound of formula I. In another embodiment, the SARM is an impurity of the compound of formula I. In another embodiment, the SARM is a prodrug of the compound of formula I. In another embodiment, the SARM is a combination of any of an analog, derivative, metabolite, isomer, pharmaceutically acceptable salt, pharmaceutical product, hydrate, *N*-oxide, crystal, impurity, polymorph or prodrug of the compound of formula 1.

In one embodiment, the SARM compound is a compound of formula I wherein X is O. In one embodiment, the SARM compound is a compound of formula I wherein G is O. In another embodiment, the SARM compound is a compound of formula I wherein Z is NO₂, In another embodiment, the SARM compound is a compound of formula I wherein Z is CN. In another embodiment, the SARM compound is a compound of formula I wherein Y is CF₃. In another embodiment, the SARM compound is a compound of formula I wherein Q is NHCOCH₃. In another embodiment, the SARM compound is a compound of formula I wherein Q is F. In another embodiment, the SARM compound is a compound of formula I wherein T is OH. In another embodiment, the SARM compound is a compound of formula I wherein R₁ is CH₃.

In another embodiment, the SARM compound useful in the methods of this invention, is characterized by the structure of formula II: 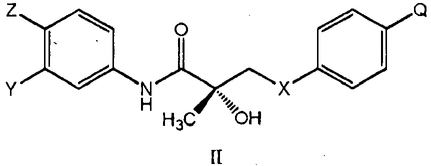
wherein X is a bond, O, CH₂, NH, Se, PR, NO or NR;
Z is NO₂, CN, COOH, COR, NHCOR or CONHR;
Y is CF₃, F, I, Br, Cl, CN, C(R)₃ or Sn(R)₃;
Q is alkyl, halogen, CF₃, CN, C(R)₃, Sn(R)₃, N(R)2., NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OR, COR, OCOR, OSO₂R, SO₂R, SR; or Q together with the benzene ring to which it is attached is a fused ring system represented by structure A, B or C: 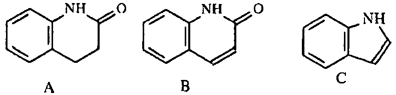 R is alkyl, haloalkyl, dihaloalkyl, trihaloalkyl, CH₂F, CHF₂, CF₃,CF₂CF₃, aryl, phenyl, halogen, alkenyl or OH.

In one embodiment, the SARM is an analog of the compound of formula II. In another embodiment, the SARM is a derivative of the compound of formula II. In another embodiment, the SARM is an isomer of the compound of formula II. In another embodiment, the SARM is a metabolite of the compound of formula II. In another embodiment, the SARM is a pharmaceutically acceptable salt of the compound of formula II. In another embodiment, the SARM is a pharmaceutical product of the compound of formula II. In another embodiment, the SARM is a hydrate of the compound of formula II. In another embodiment, the SARM is an *N*-oxide of the compound of formula II. In another embodiment, the SARM is a crystal of the compound of formula II. In another embodiment, the SARM is a polymorph of the compound of formula II. In another embodiment, the SARM is an impurity of the compound of formula II. In another embodiment, the SARM is a prodrug of the compound of formula II: In another embodiment, the SARM is a prodrug of the compound of formula II. In another embodiment, the SARM is a combination of any of an analog, derivative, metabolite, isomer, pharmaceutically acceptable salt, pharmaceutical product, hydrate, *N*-oxide, crystal, polymorph, impurity or prodrug of the compound of formula II.

In one embodiment, the SARM compound is a compound of formula II wherein X is O. In another embodiment, the SARM compound is a compound of formula II wherein Z is NO₂. In another embodiment, the SARM compound is a compound of formula II wherein Z is CN. In another embodiment, the SARM compound is a compound of formula II wherein Y is CF₃. In another embodiment, the SARM compound is a compound of formula II wherein Q is NHCOCH₃. In another embodiment, the SARM compound is a compound of formula II wherein Q is F. In another embodiment, the SARM compound is a compound of formula II where Q is halogen, i.e. F, Cl, Br or I.

In another embodiment, the SARM compound is a compound of formula II wherein X is O, Z is NO₂, Y is CF₃ and Q is halogen. In another embodiment, the SARM compound is a compound of formula II wherein X is O, Z is NO₂ Y is CF₃ and Q is NHCOCH₃. In another embodiment, the SARM compound is a compound of formula II wherein X is O, Z is CN, Y is CF₃ and Q is halogen. In another embodiment, the SARM compound is a compound of formula II wherein X is O, Z is CN, Y is CF₃ and Q is NHCOCH₃.

In another embodiment, the SARM compound useful in the methods of this invention, is characterized by the structure of formula III: 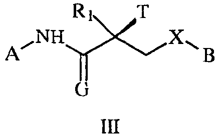
wherein X is a bond, O, CH₂, NH, Se, PR, NO or NR;
G is O or S;
R₁ is CH₃, CH₂F, CHF₂, CF₃, CH₂CH₃, or CF₂CF₃;
T is OH, OR, -NHCOCH₃, or NHCOR;
R is alkyl, haloalkyl, dihaloalkyl, trihaloalkyl, CH₂F, CHF₂, CF₃, CF₂CF₃, aryl, phenyl, halogen, alkenyl or OH;
A is a ring selected from: 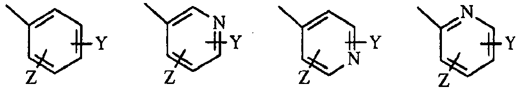 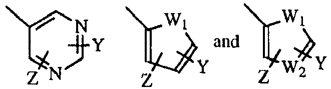
B is a ring selected from: 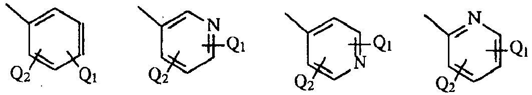 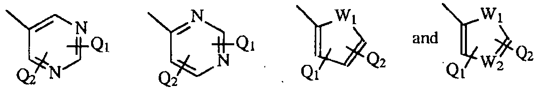
   wherein A and B cannot simultaneously be a benzene ring;
   Z is NO₂, CN, COOH, COR, NHCOR or CONHR;
   Y is CF₃, F, I, Br, Cl, CN CR₃ or Sn(R)₃;
   Q₁ and Q₂ are independently of each other a hydrogen, alkyl, halogen, CF₃, CN C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OR, COR, OCOR, OSO₂R, SO₂R, SR, 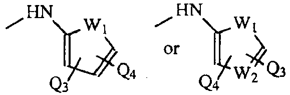
      Q₃ and Q₄ are independently of each other a hydrogen, alkyl, halogen, CF₃, CN C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OR, COR, OCOR, OSO₂R, SO₂R or SR;
      W₁ is O, NH, NR, NO or S; and
      W₂ is N or NO.

In one embodiment, the SARM is an analog of the compound of formula III. In another embodiment, the SARM is a derivative of the compound of formula III. In another embodiment, the SARM is an isomer of the compound of formula III. In another embodiment, the SARM is a metabolite of the compound of formula III. In another embodiment, the SARM is a pharmaceutically acceptable salt of the compound of formula III. In another embodiment, the SARM is a pharmaceutical product of the compound of formula III. In another embodiment, the SARM is a hydrate of the compound of formula III. In another embodiment, the SARM is an N-oxide of the compound of formula III. In another embodiment, the SARM is a crystal of the compound of formula III. In another embodiment, the SARM is a polymorph of the compound of formula III. In another embodiment, the SARM is a prodrug of the compound of formula III. In another embodiment, the SARM is an impurity of the compound of formula III. In another embodiment, the SARM is a combination of any of an analog, derivative, metabolite, isomer, pharmaceutically acceptable salt, pharmaceutical product, hydrate, *N-*oxide, crystal, polymorph, impurity or prodrug of the compound of formula III.

In one embodiment, the SARM compound is a compound of formula III wherein X is O. In another embodiment, the SARM compound is a compound of formula III wherein G is O. In another embodiment, the SARM compound is a compound of formula I wherein T is OH. In another embodiment, the SARM compound is a compound of formula III wherein R₁ is CH₃. In another embodiment, the SARM compound is a compound of formula III wherein Z is NO₂. In another embodiment, the SARM compound is a compound of formula III wherein Z is CN. In another embodiment, the SARM compound is a compound of formula III wherein Y is CF₃. In another embodiment, the SARM compound is a compound of formula III wherein Q₁ is NHCOCH₃. In another embodiment, the SARM compound is a compound of formula III wherein Q₁ is F.

The substituents Z and Y can be in any position of the ring carrying these substituents (hereinafter "A ring"). In one embodiment, the substituent Z is in the para position of the A ring. In another embodiment, the substituent Y is in the meta position of the A ring. In another embodiment, the substituent Z is in the para position of the A ring and substituent Y is in the meta position of the A ring.

The substituents Q₁ and Q₂ can be in any position of the ring carrying these substituents (hereinafter "B ring"). In one embodiment, the substitutent Q₁ is in the para position of the B ring. In another embodiment, the subsituent is Q₂ is H. In another embodiment, the substituted Q₁ is in the para position of the B ring and the subsituent is Q₂ is H. In another embodiment, the substitutent Q₁ is NHCOCH₃ and is in the para position of the B ring, and the substituent is Q₂ is H.

In another embodiment, the SARM compound useful in the methods of this invention, is characterized by the structure of formula IV: 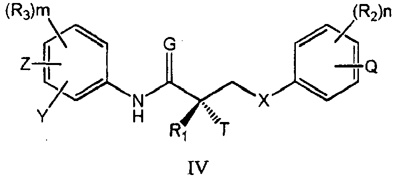
wherein X is a bond, O, CH₂, NH, Se, PR, NO or NR;
G is O or S;
T is OH, OR, -NHCOCH₃, or NHCOR;
R is alkyl, haloalkyl, dihaloalkyl, trihaloalkyl, CH₂F, CHF₂, CF₃, CF₂CF₃, aryl, phenyl, halogen, alkenyl or OH;
R₁ is CH₃, CH₂F, CHF₂, CF₃, CH₂CH₃, or CF₂CF₃;
R₂ is F, Cl, Br, I, CH₃, CF₃, OH, CN, NO₂, NHCOCH₃, NHCOCF₃, NHCOR, alkyl, arylalkyl, OR, NH₂, NHR, N(R)₂, SR;
R₃ is F, Cl, Br, I, CN, NO₂, COR, COOH, CONHR, CF₃, Sn(R)₃, or R₃ together with the benzene ring to which it is attached forms a fused ring system represented by the structure: 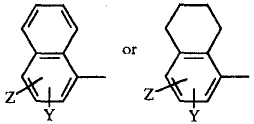
   Z is NO₂, CN, COR, COOH, or CONHR;
   Y is CF₃, F, Br, Cl, I, CN, or Sn(R)₃;
   Q is H, alkyl, halogen, CF₃, CN, C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OH, OR, COR, OCOR, OSO₂R, SO₂R, SR; or Q together with the benzene ring to which it is attached is a fused ring system represented by structure A, B or C: 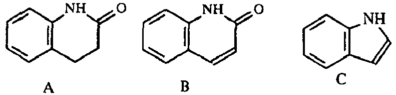
      n is an integer of 1-4; and
      m is an integer of 1-3.

In one embodiment, the SARM is an analog of the compound of formula IV. In another embodiment, the SARM is a derivative of the compound of formula IV. In another embodiment, the SARM is an isomer of the compound of formula IV. In another embodiment, the SARM is a metabolite of the compound of formula IV. In another embodiment, the SARM is a pharmaceutically acceptable salt of the compound of formula IV. In another embodiment, the SARM is a pharmaceutical product of the compound of formula IV. In another embodiment, the SARM is a hydrate of the compound of formula IV. In another embodiment, the SARM is an *N*-oxide of the compound of formula IV. In another embodiment, the SARM is a crystal of the compound of formula IV. In another embodiment, the SARM is a polymorph of the compound of formula IV. In another embodiment, the SARM is a prodrug of the compound of formula IV. In another embodiment, the SARM is an impurity of the compound of formula IV. In another embodiment, the SARM is a combination of any of an analog, derivative, metabolite, isomer, pharmaceutically acceptable salt, pharmaceutical product, hydrate, *N-*oxide, crystal, polymorph, impurity or prodrug of the compound of formula IV.

In one embodiment, the SARM compound is a compound of formula IV wherein X is O. In another embodiment, the SARM compound is a compound of formula IV wherein G is O. In another embodiment, the SARM compound is a compound of formula IV wherein Z is NO₂. In another embodiment, the SARM compound is a compound of formula IV wherein Z is CN. In another embodiment, the SARM compound is a compound of formula IV wherein Y is CF₃. In another embodiment, the SARM compound is a compound of formula IV wherein Q is NHCOCH₃. In another embodiment, the SARM compound is a compound of formula IV wherein Q is F. In another embodiment, the SARM compound is a compound of formula IV wherein T is OH. In another embodiment, the SARM compound is a compound of formula IV wherein R₁ is CH₃. In another embodiment, the SARM compound is a compound of formula IV wherein Q is F and R₂ is CH₃. In another embodiment, the SARM compound is a compound of formula IV wherein Q is F and R₂ is Cl.

The substituents Z, Y and R₃ can be in any position of the ring carrying these substituents (hereinafter "A ring"). In one embodiment, the substituent Z is in the para position of the A ring. In another embodiment, the substituent Y is in the meta position of the A ring. In another embodiment, the substituent Z is in the para position of the A ring and substituent Y is in the meta position of the A ring.

The substituents Q and R₂ can be in any position of the ring carrying these substituents (hereinafter "B ring"). In one embodiment, the substitutent Q is in the para position of the B ring. In another embodiment, the substitutent Q is in the para position of the B ring. In another embodiment, the substitutent Q is NHCOCH₃ and is in the para position of the B ring.

As contemplated herein, when the integers m and n are greater than one, the substituents R₂ and R₃ are not limited to one particular substituent, and can be any combination of the substituents listed above.

In another embodiment, the SARM compound which is effective in the methods of this invention, is characterized by the structure of formula V:

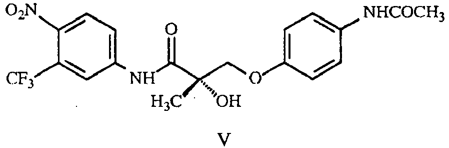

In one embodiment, the SARM is an analog of the compound of formula V. In another embodiment, the SARM is a derivative of the compound of formula V. In another embodiment, the SARM is an isomer of the compound of formula V. In another embodiment, the SARM is a metabolite of the compound of formula V. In another embodiment, the SARM is a pharmaceutically acceptable salt of the compound of formula V. In another embodiment, the SARM is a pharmaceutical product of the compound of formula V. In another embodiment, the SARM is a hydrate of the compound of formula V. In another embodiment, the SARM is an *N*-oxide of the compound of formula V. In another embodiment, the SARM is a crystal of the compound of formula V. In another embodiment, the SARM is a polymorph of the compound of formula V. In another embodiment, the SARM is an impurity of the compound of formula V. In another embodiment, the SARM is a prodrug of the compound of formula V. In another embodiment, the SARM is a combination of any of an analog, derivative, metabolite, isomer, pharmaceutically acceptable salt, pharmaceutical product, hydrate, *N-*oxide, crystal, polymorph, impurity or prodrug of the compound of formula V.

In another embodiment, the SARM compound which is effective in the methods of this invention, is characterized by the structure of formula VI:

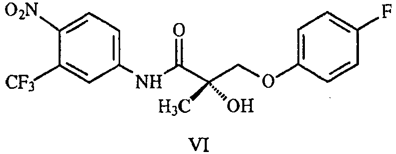

In one embodiment, the SARM is an analog of the compound of formula VI. In another embodiment, the SARM is a derivative of the compound of formula VI. In another embodiment, the SARM is an isomer of the compound of formula VI. In another embodiment, the SARM is a metabolite of the compound of formula VI. In another embodiment, the SARM is a pharmaceutically acceptable salt of the compound of formula VI. In another embodiment, the SARM is a pharmaceutical product of the compound of formula VI. In another embodiment, the SARM is a hydrate of the compound of formula VI. In another embodiment, the SARM is an *N*-oxide of the compound of formula VI. In another embodiment, the SARM is a crystal of the compound of formula VI. In another embodiment, the SARM is a polymorph of the compound of formula VI. In another embodiment, the SARM is an impurity of the compound of formula VI. In another embodiment, the SARM is a prodrug of the compound of formula VI. In another embodiment, the SARM is a combination of any of an analog, derivative, metabolite, isomer, pharmaceutically acceptable salt, pharmaceutical product, hydrate, *N-*oxide, crystal, polymorph, impurity or prodrug of the compound of formula VI.

In another embodiment, the SARM compound which is effective in the methods of this invention is characterized by the structure of formula VII:

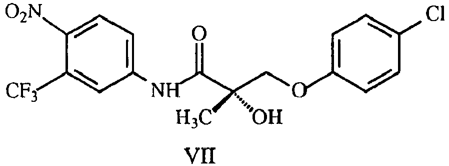

In one embodiment, the SARM is an analog of the compound of formula VII. In another embodiment, the SARM is a derivative of the compound of formula VII. In another embodiment, the SARM is an isomer of the compound of formula VII. In another embodiment, the SARM is a metabolite of the compound of formula VII. In another embodiment, the SARM is a pharmaceutically acceptable salt of the compound of formula VII. In another embodiment, the SARM is a pharmaceutical product of the compound of formula VII. In another embodiment, the SARM is a hydrate of the compound of formula VII. In another embodiment, the SARM is an *N*-oxide of the compound of formula VII. In another embodiment, the SARM is a crystal of the compound of formula VII. In another embodiment, the SARM is a polymorph of the compound of formula VII. In another embodiment, the SARM is an impurity of the compound of formula VII. In another embodiment, the SARM is a prodrug of the compound of formula VII. In another embodiment, the SARM is a combination of any of an analog, derivative, metabolite, isomer, pharmaceutically acceptable salt, pharmaceutical product, hydrate, *N-*oxide, crystal, polymorph, impurity or prodrug of the compound of formula VII.

In another embodiment, the SARM compound which is effective in the methods of this invention is characterized by the structure of formula VIII:

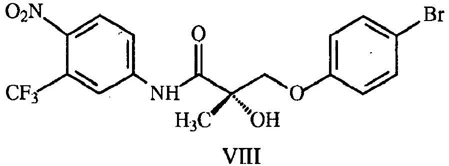

In one embodiment, the SARM is an analog of the compound of formula VIII In another embodiment, the SARM is a derivative of the compound of formula VIII. In another embodiment, the SARM is an isomer of the compound of formula VIII. In another embodiment, the SARM is a metabolite of the compound of formula VIII. In another embodiment, the SARM is a pharmaceutically acceptable salt of the compound of formula VIII. In another embodiment, the SARM is a pharmaceutical product of the compound of formula VIII. In another embodiment, the SARM is a hydrate of the compound of formula VIII. In another embodiment, the SARM is an *N*-oxide of the compound of formula VIII. In another embodiment, the SARM is a crystal of the compound of formula VIII. In another embodiment, the SARM is a polymorph of the compound of formula VIII. In another embodiment, the SARM is an impurity of the compound of formula VIII. In another embodiment, the SARM is a prodrug of the compound of formula VIII. In another embodiment, the SARM is a combination of any of an analog, derivative, metabolite, isomer, pharmaceutically acceptable salt, pharmaceutical product, hydrate, *N*-oxide, crystal, polymorph, impurity or prodrug of the compound of formula VIII.

In another embodiment, the SARM compound which is effective in the methods of this invention is characterized by the structure of formula IX:

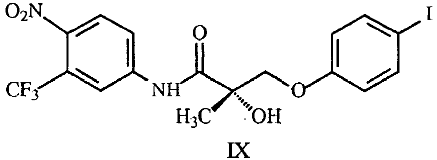

In one embodiment, the SARM is an analog of the compound of formula IX. In another embodiment, the SARM is a derivative of the compound of formula IX. In another embodiment, the SARM is an isomer of the compound of formula IX. In another embodiment, the SARM is a metabolite of the compound of formula IX. In another embodiment, the SARM is a pharmaceutically acceptable salt of the compound of formula IX. In another embodiment, the SARM is a pharmaceutical product of the compound of formula IX. In another embodiment, the SARM is a hydrate of the compound of formula IX. In another embodiment, the SARM is an *N*-oxide of the compound of formula IX. In another embodiment, the SARM is a crystal of the compound of formula IX. In another embodiment, the SARM is a polymorph of the compound of formula IX. In another embodiment, the SARM is an impurity of the compound of formula IX. In another embodiment, the SARM is a prodrug of the compound of formula IX. In another embodiment, the SARM is a combination of any of an analog, derivative, metabolite, isomer, pharmaceutically acceptable salt, pharmaceutical product, hydrate, *N-*oxide, crystal, polymorph, impurity or prodrug of the compound of formula IX.

In another embodiment, the SARM compound which is effective in the methods of this invention is characterized by the structure of formula X:

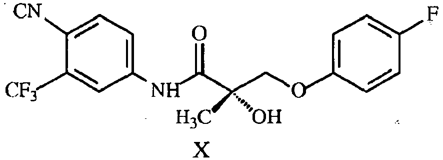

In one embodiment, the SARM is an analog of the compound of formula X. In another embodiment, the SARM is a derivative of the compound of formula X. In another embodiment, the SARM is an isomer of the compound of formula X. In another embodiment, the SARM is a metabolite of the compound of formula X. In another embodiment, the SARM is a pharmaceutically acceptable salt of the compound of formula X. In another embodiment, the SARM is a pharmaceutical product of the compound of formula X. In another embodiment, the SARM is a hydrate of the compound of formula X. In another embodiment, the SARM is an *N*-oxide of the compound of formula X. In another embodiment, the SARM is a crystal of the compound of formula X. In another embodiment, the SARM is a polymorph of the compound of formula X. In another embodiment, the SARM is an impurity of the compound of formula X. In another embodiment, the SARM is a prodrug of the compound of formula X. In another embodiment, the SARM is a combination of any of an analog, derivative, metabolite, isomer, pharmaceutically acceptable salt, pharmaceutical product, hydrate, *N-*oxide, crystal, polymorph, impurity or prodrug of the compound of formula X.

In another embodiment, the SARM compound which is effective in the methods of this invention is characterized by the structure of formula XI:

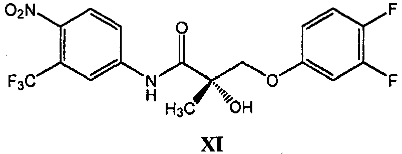

In one embodiment, the SARM is an analog of the compound of formula XI. In another embodiment, the SARM is a derivative of the compound of formula XI. In another embodiment, the SARM is an isomer of the compound of formula XI. In another embodiment, the SARM is a metabolite of the compound of formula XI. In another embodiment, the SARM is a pharmaceutically acceptable salt of the compound of formula XI. In another embodiment, the SARM is a pharmaceutical product of the compound of formula XI. In another embodiment, the SARM is a hydrate of the compound of formula XI. In another embodiment, the SARM is an N-oxide of the compound of formula XI. In another embodiment, the SARM is a crystal of the compound of formula XI. In another embodiment, the SARM is a polymorph of the compound of formula XI. In another embodiment, the SARM is an impurity of the compound of formula XI. In another embodiment, the SARM is a prodrug of the compound of formula XI. In another embodiment, the SARM is a combination of any of an analog, derivative, metabolite, isomer, pharmaceutically acceptable salt, pharmaceutical product, hydrate, *N-*oxide, crystal, polymorph, impurity or prodrug of the compound of formula XI.

In another embodiment, the SARM compound which is effective in the methods of this invention is characterized by the structure of formula XII:

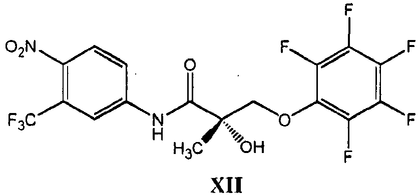

In one embodiment, the SARM is an analog of the compound of formula XII. In another embodiment, the SARM is a derivative of the compound of formula XII. In another embodiment, the SARM is an isomer of the compound of formula XII. In another embodiment, the SARM is a metabolite of the compound of formula XII. In another embodiment, the SARM is a pharmaceutically acceptable salt of the compound of formula XII. In another embodiment, the SARM is a pharmaceutical product of the compound of formula XII. In another embodiment, the SARM is a hydrate of the compound of formula XII. In another embodiment, the SARM is an *N*-oxide of the compound of formula XII. In another embodiment, the SARM is a crystal of the compound of formula XII. In another embodiment, the SARM is a polymorph of the compound of formula XII. In another embodiment, the SARM is an impurity of the compound of formula XII. In another embodiment, the SARM is a prodrug of the compound of formula XII. In another embodiment, the SARM is a combination of any of an analog, derivative, metabolite, isomer, pharmaceutically acceptable salt, pharmaceutical product, hydrate, *N-*oxide, crystal, polymorph, impurity or prodrug of the compound of formula XII.

In another embodiment, the SARM compound which is effective in the methods of this invention is characterized by the structure of formula XIII: 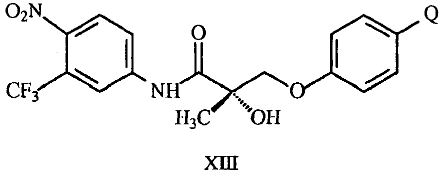 wherein Q is as defined above for formula I.

In one embodiment, the SARM is an analog of the compound of formula XIII. In another embodiment, the SARM is a derivative of the compound of formula XIII. In another embodiment, the SARM is an isomer of the compound of formula XIII. In another embodiment, the SARM is a metabolite of the compound of formula XIII. In another embodiment, the SARM is a pharmaceutically acceptable salt of the compound of formula XIII. In another embodiment, the SARM is a pharmaceutical product of the compound of formula XIII. In another embodiment, the SARM is a hydrate of the compound of formula XIII. In another embodiment, the SARM is an *N*-oxide of the compound of formula XIII. In another embodiment, the SARM is a crystal of the compound of formula XIII. In another embodiment, the SARM is a polymorph of the compound of formula XIII. In another embodiment, the SARM is an impurity of the compound of formula XIII. In another embodiment, the SARM is a prodrug of the compound of formula XIII. In another embodiment, the SARM is a combination of any of an analog, derivative, metabolite, isomer, pharmaceutically acceptable salt, pharmaceutical product, hydrate, *N*-oxide, crystal, polymorph, impurity or prodrug of the compound of formula XIII.

In one embodiment, the SARM is a compound of formula XIII wherein Q is F. In another embodiment, the SARM is a compound of formula XIII wherein Q is Cl. In another embodiment, the SARM is a compound of formula XIII wherein Q is Br. In another embodiment, the SARM is a compound of formula XIII wherein Q is 1. In another embodiment, the SARM is a compound of formula XIII wherein Q is NHCOCH₃.

In another embodiment, the SARM compound which is effective in the methods of this invention is characterized by the structure of formula XIV: 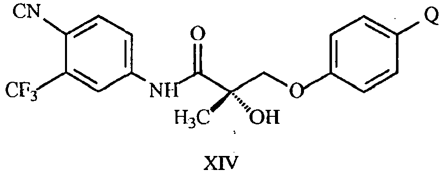 wherein Q is as defined above for formula I.

In one embodiment, the SARM is an analog of the compound of formula XIV. In another embodiment, the SARM is a derivative of the compound of formula XIV. In another embodiment, the SARM is an isomer of the compound of formula XIV. In another embodiment, the SARM is a metabolite of the compound of formula XIV. In another embodiment, the SARM is a pharmaceutically acceptable salt of the compound of formula XIV. In another embodiment, the SARM is a pharmaceutical product of the compound of formula XIV. In another embodiment, the SARM is a hydrate of the compound of formula XIV. In another embodiment, the SARM is an *N*-oxide of the compound of formula XIV. In another embodiment, the SARM is a crystal of the compound of formula XIV. In another embodiment, the SARM is a polymorph of the compound of formula XIV. In another embodiment, the SARM is an impurity of the compound of formula XIV. In another embodiment, the SARM is a prodrug of the compound of formula XIV. In another embodiment, the SARM is a combination of any of an analog, derivative, metabolite, isomer, pharmaceutically acceptable salt, pharmaceutical product, hydrate, *N*-oxide, crystal, polymorph, impurity or prodrug of the compound of formula XIV.

In one embodiment, the SARM is a compound of formula XIV wherein Q is F. In another embodiment, the SARM is a compound of formula XIV wherein Q is Cl. In another embodiment, the SARM is a compound of formula XIV wherein Q is Br. In another embodiment, the SARM is a compound of formula XIV wherein Q is 1. In another embodiment, the SARM is a compound of formula XIV wherein Q is NHCOCH₃.

In another embodiment, the SARM compound which is effective in the methods of this invention is characterized by the structure of formula XV:

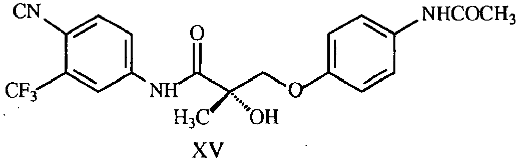

In one embodiment, the SARM is an analog of the compound of formula XV. In another embodiment, the SARM is a derivative of the compound of formula XV. In another embodiment, the SARM is an isomer of the compound of formula XV. In another embodiment, the SARM is a metabolite of the compound of formula XV. In another embodiment, the SARM is a pharmaceutically acceptable salt of the compound of formula XV. In another embodiment, the SARM is a pharmaceutical product of the compound of formula XV. In another embodiment, the SARM is a hydrate of the compound of formula XV. In another embodiment, the SARM is an *N*-oxide of the compound of formula XV. In another embodiment, the SARM is a crystal of the compound of formula XV. In another embodiment, the SARM is a polymorph of the compound of formula XV. In another embodiment, the SARM is an impurity of the compound of formula XV. In another embodiment, the SARM is a prodrug of the compound of formula XV. In another embodiment, the SARM is a combination of any of an analog, derivative, metabolite, isomer, pharmaceutically acceptable salt, pharmaceutical product, hydrate, *N*-oxide, crystal, polymorph, impurity or prodrug of the compound of formula XV.

In another embodiment, the SARM compound which is effective in the methods of this invention is characterized by the structure of formula XVI:

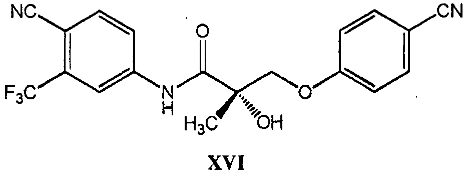

In one embodiment, the SARM is an analog of the compound of formula XVI. In another embodiment, the SARM is a derivative of the compound of formula XVI. In another embodiment, the SARM is an isomer of the compound of formula XVI. In another embodiment, the SARM is a metabolite of the compound of formula XVI. In another embodiment, the SARM is a pharmaceutically acceptable salt of the compound of formula XVI. In another embodiment, the SARM is a pharmaceutical product of the compound of formula XVI. In another embodiment, the SARM is a hydrate of the compound of formula XVI. In another embodiment, the SARM is an N-oxide of the compound of formula XVI. In another embodiment, the SARM is a crystal of the compound of formula XVI. In another embodiment, the SARM is a polymorph of the compound of formula XVI. In another embodiment, the SARM is an impurity of the compound of formula XVI. In another embodiment, the SARM is a prodrug of the compound of formula XVI. In another embodiment, the SARM is a combination of any of an analog, derivative, metabolite, isomer, pharmaceutically acceptable salt, pharmaceutical product, hydrate, N-oxide, crystal, polymorph, impurity or prodrug of the compound of formula XVI.

In another embodiment, the SARM compound which is effective in the methods of this invention is characterized by the structure of formula XVII:

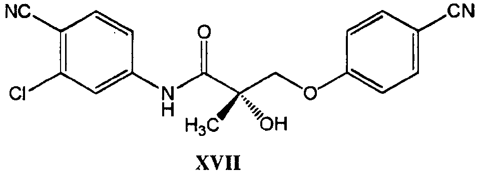

In one embodiment, the SARM is an analog of the compound of formula XVII. In another embodiment, the SARM is a derivative of the compound of formula XVII. in another embodiment, the SARM is an isomer of the compound of formula XVII. In another embodiment, the SARM is a metabolite of the compound of formula XVII. In another embodiment, the SARM is a pharmaceutically acceptable salt of the compound of formula XVII. In another embodiment, the SARM is a pharmaceutical product of the compound of formula XVII. In another embodiment, the SARM is a hydrate of the compound of formula XVII. In another embodiment, the SARM is an *N*-oxide of the compound of formula XVII. In another embodiment, the SARM is a crystal of the compound of formula XVII. In another embodiment, the SARM is a polymorph of the compound of formula XVII. In another embodiment, the SARM is an impurity of the compound of formula XVII. In another embodiment, the SARM is a prodrug of the compound of formula XVII. In another embodiment, the SARM is a combination of any of an analog, derivative, metabolite, isomer, pharmaceutically acceptable salt, pharmaceutical product, hydrate, *N*-oxide, crystal, polymorph, impurity or prodrug of the compound of formula XVII.

In another embodiment, the SARM compound which is effective in the methods of this invention is characterized by the structure of formula XVIII:

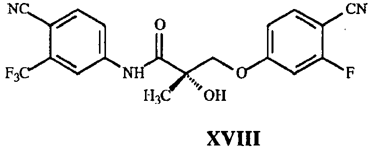

In one embodiment, the SARM is an analog of the compound of formula XVIII. In another embodiment, the SARM is a derivative of the compound of formula XVIII. In another embodiment, the SARM is an isomer of the compound of formula XVIII. In another embodiment, the SARM is a metabolite of the compound of formula XVIII. In another embodiment, the SARM is a pharmaceutically acceptable salt of the compound of formula XVIII. In another embodiment, the SARM is a pharmaceutical product of the compound of formula XVIII. In another embodiment, the SARM is a hydrate of the compound of formula XVIII. In another embodiment, the SARM is an *N*-oxide of the compound of formula XVIII. In another embodiment, the SARM is a crystal of the compound of formula XVIII. In another embodiment, the SARM is a polymorph of the compound of formula XVIII. In another embodiment, the SARM is an impurity of the compound of formula XVIII. In another embodiment, the SARM is a prodrug of the compound of formula XVIII. In another embodiment, the SARM is a combination of any of an analog, derivative, metabolite, isomer, pharmaceutically acceptable salt, pharmaceutical product, hydrate, N-oxide, crystal, polymorph, impurity or prodrug of the compound of formula XVIII.

In another embodiment, the SARM compound which is effective in the methods of this invention is characterized by the structure of formula XIX:

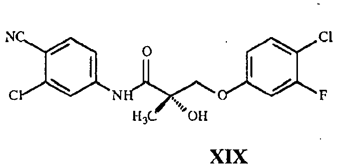

In one embodiment, the SARM is an analog of the compound of formula XIX. In another embodiment, the SARM is a derivative of the compound of formula XIX. In another embodiment, the SARM is an isomer of the compound of formula XIX. In another embodiment, the SARM is a metabolite of the compound of formula XIX. In another embodiment, the SARM is a pharmaceutically acceptable salt of the compound of formula XIX. In another embodiment, the SARM is a pharmaceutical product of the compound of formula XIX. In another embodiment, the SARM is a hydrate of the compound of formula XIX. In another embodiment, the SARM is an *N*-oxide of the compound of formula XIX. In another embodiment, the SARM is a crystal of the compound of formula XIX. In another embodiment, the SARM is a polymorph of the compound of formula XIX. In another embodiment, the SARM is an impurity of the compound of formula XIX. In another embodiment, the SARM is a prodrug of the compound of formula XIX. In another embodiment, the SARM is a combination of any of an analog, derivative, metabolite, isomer, pharmaceutically acceptable salt, pharmaceutical product, hydrate, *N*-oxide, crystal, polymorph, impurity or prodrug of the compound of formula XIX.

In another embodiment, the SARM compound which is effective in the methods of this invention is characterized by the structure of formula XX:

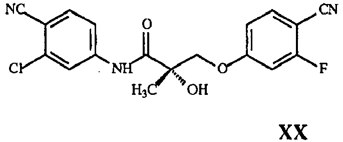

In one embodiment, the SARM is an analog of the compound of formula XX. In another embodiment, the SARM is a derivative of the compound of formula XX. In another embodiment, the SARM is an isomer of the compound of formula XX. In another embodiment, the SARM is a metabolite of the compound of formula XX. In another embodiment, the SARM is a pharmaceutically acceptable salt of the compound of formula XX. In another embodiment, the SARM is a pharmaceutical product of the compound of formula XX. In another embodiment, the SARM is a hydrate of the compound of formula XX. In another embodiment, the SARM is an *N*-oxide of the compound of formula XX. In another embodiment, the SARM is a crystal of the compound of formula XX. In another embodiment, the SARM is a polymorph of the compound of formula XX. In another embodiment, the SARM is an impurity of the compound of formula XX. In another embodiment, the SARM is a prodrug of the compound of formula XX In another embodiment, the SARM is a combination of any of an analog, derivative, metabolite, isomer, pharmaceutically acceptable salt, pharmaceutical product, hydrate, *N*-oxide, crystal, polymorph, impurity or prodrug of the compound of formula XX.

The substituent R in the SARM compounds of the present invention is defined herein as an alkyl, haloalkyl, dihaloalkyl, trihaloalkyl, CH₂F, CHF₂, CF₃, CF₂CF₃, aryl, phenyl, halogen, alkenyl or OH.

An "alkyl" group refers to a saturated aliphatic hydrocarbon, including straight-chain, branched-chain and cyclic alkyl groups. In one embodiment, the alkyl group has 1-12 carbons. In another embodiment, the alkyl group has 1-7 carbons. In another embodiment, the alkyl group has 1-6 carbons. In another embodiment, the alkyl group has 1-4 carbons. The alkyl group may be unsubstituted or substituted by one or more groups selected from halogen, hydroxy, alkoxy carbonyl, amido, alkylamido, dialkylamido, nitro, amino, alkylamino, dialkylamino, carboxyl, thio and thioalkyl.

An "alkenyl" group refers to an unsaturated hydrocarbon, including straight chain, branched chain and cyclic groups having one or more double bond. The alkenyl group may have one double bond, two double bonds, three double bonds etc. Examples of alkenyl groups are ethenyl, propenyl, butenyl, cyclohexenyl etc. The alkenyl group may be unsubstituted or substituted by one or more groups selected from halogen, hydroxy, alkoxy carbonyl, amido, alkylamido, dialkylamido, nitro, amino, alkylamino, dialkylamino, carboxyl, thio and thioalkyl.

A "haloalkyl" group refers to an alkyl group as defined above, which is substituted by one or more halogen atoms, e.g. by F, Cl, Br or I.

An "aryl" group refers to an aromatic group having at least one carbocyclic aromatic group or heterocyclic aromatic group, which may be unsubstituted or substituted by one or more groups selected from halogen, haloalkyl, hydroxy, alkoxy carbonyl, amido, alkylamido, dialkylamido, nitro, amino, alkylamino, dialkylamino, carboxy or thio or thioalkyl. Nonlimiting examples of aryl rings are phenyl, naphthyl, pyranyl, pyrrolyl, pyrazinyl, pyrimidinyl, pyrazolyl, pyridinyl, furanyl, thiophenyl, thiazolyl, imidazolyl, isoxazolyl, and the like.

A "hydroxyl" group refers to an OH group. It is understood by a person skilled in the art that when T in the compounds of the present invention is OR, R is not OH. A halo group refers to F, Cl, Br or I.

An "arylalkyl" group refers to an alkyl bound to an aryl, wherein alkyl and aryl are as defined above. An example of an arylalkyl group is a benzyl group.

As contemplated herein, the present invention relates to the use of a SARM compound and/or an analog, derivative, isomer, metabolite, pharmaceutically acceptable salt, pharmaceutical product, hydrate, N-oxide, prodrug, polymorph, impurity or crystal or combinations thereof. In one embodiment, the invention relates to the use of an analog of the SARM compound. In another embodiment, the invention relates to the use of a derivative of the SARM compound. In another embodiment, the invention relates to the use of an isomer of the SARM compound. In another embodiment, the invention relates to the use of a metabolite of the SARM compound. In another embodiment, the invention relates to the use of a pharmaceutically acceptable salt of the SARM compound. In another embodiment, the invention relates to the use of a pharmaceutical product of the SARM compound. In another embodiment, the invention relates to the use of a hydrate of the SARM compound. In another embodiment, the invention relates to the use of an *N*-oxide of the SARM compound. In another embodiment, the invention relates to the use of a prodrug of the SARM compound. In another embodiment, the invention relates to the use of a polymorph of the SARM compound. In another embodiment, the invention relates to the use of a crystal of the SARM compound. In another embodiment, the invention relates to the use of an impurity of the SARM compound. In another embodiment, the invention relates to the use of any of a combination of an analog, derivative, isomer, metabolite, pharmaceutically acceptable salt, pharmaceutical product, hydrate, *N*-oxide, prodrug, polymorph, impurity or crystal of the SARM compounds of the present invention.

In one embodiment, the term "isomer" includes, but is not limited to, optical isomers and analogs, structural isomers and analogs, conformational isomers and analogs, and the like.

In one embodiment, this invention encompasses the use of various optical isomers of the SARM compound. It will be appreciated by those skilled in the art that the SARMs of the present invention contain at least one chiral center. Accordingly, the SARMs used in the methods of the present invention may exist in, and be isolated in, optically-active or racemic forms. Some compounds may also exhibit polymorphism. It is to be understood that the present invention encompasses any racemic, optically-active, polymorphic, or stereroisomeric form, or mixtures thereof, which form possesses properties useful in the treatment of conditions described herein. In one embodiment, the SARMs are the pure (R)-isomers. In another embodiment, the SARMs are the pure (S)-isomers. In another embodiment, the SARMs are a mixture of the (R) and the (S) isomers. In another embodiment, the SARMs are a racemic mixture comprising an equal amount of the (R) and the (S) isomers. It is well known in the art how to prepare optically-active forms (for example, by resolution of the racemic form by recrystallization techniques, by synthesis from optically-active starting materials, by chiral synthesis, or by chromatographic separation using a chiral stationary phase).

The invention includes "pharmaceutically acceptable salts" of amino-substituted compounds with organic and inorganic acids, for example, citric acid and hydrochloric acid. The invention also includes *N-*oxides of the amino substituents of the compounds described herein. Pharmaceutically acceptable salts can also be prepared from the phenolic compounds by treatment with inorganic bases, for example, sodium hydroxide. Also, esters of the phenolic compounds can be made with aliphatic and aromatic carboxylic acids, for example, acetic acid and benzoic acid esters.

This invention further includes derivatives of the SARM compounds. In one embodiment, "derivatives" includes but is not limited to ether derivatives, acid derivatives, amide derivatives, ester derivatives and the like. In addition, this invention further includes hydrates of the SARM compounds. In one embodiment, "hydrate" includes but is not limited to hemihydrate, monohydrate, dihydrate, trihydrate and the like.

This invention further includes metabolites of the SARM compounds. In one embodiment, "metabolite" means any substance produced from another substance by metabolism or a metabolic process.

This invention further includes pharmaceutical products of the SARM compounds. The term "pharmaceutical product" means a composition suitable for pharmaceutical use (pharmaceutical composition), as defined herein.

### Compositions for use in the methods of this invention

In one embodiment, the SARM and any other compounds for administration according to the methods of this invention, may be in a composition. The composition, in some embodiments comprise pharmaceutically-acceptable carriers.

Some examples of substances which can serve as pharmaceutically-acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the Tween™ brand emulsifiers; wetting agents, such sodium lauryl sulfate; coloring agents; flavoring agents; tableting agents, stabilizers; antioxidants; preservatives; pyrogen-free water; isotonic saline; and phosphate buffer solutions. The choice of a pharmaceutically-acceptable carrier to be used in conjunction with the compound is basically determined by the way the compound is to be administered. If the subject compound is to be injected, the preferred pharmaceutically-acceptable carrier is sterile, physiological saline, with a blood-compatible suspending agent, the pH of which has been adjusted to about 7.4.

If the preferred mode of administering the subject compound is perorally, in one embodiment, a unit dosage form used may comprise tablets, capsules, lozenges, chewable tablets, and the like. Such unit dosage forms comprise a safe and effective amount of the desired compound, or compounds, each of which is in one embodiment, from about 0.7 or 3.5 mg to about 280 mg/70 kg, or in another embodiment, about 0.5 or 10 mg to about 210 mg/70 kg. The pharmaceutically-acceptable carrier suitable for the preparation of unit dosage forms for peroral administration are well-known in the art. Tablets typically comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. Glidants such as silicon dioxide can be used to improve flow characteristics of the powder-mixture. Coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more sol id diluents disclosed above. The selection of carrier components depends on secondary considerations like taste, cost, and shelf stability, which are not critical for the purposes of this invention, and can be readily made by a person skilled in the art.

Peroral compositions may comprise liquid solutions, emulsions, suspensions, and the like. The pharmaceutically-acceptable carriers suitable for preparation of such compositions are well known in the art. Such liquid oral compositions comprise, in some embodiments, from about 0.012% to about 0.933% of the desired compound or compounds, or in another embodiment, from about 0.033% to about 0.7%. Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, cellulose (e.g. Avicel™, RC-591), tragacanth and sodium alginate; typical wetting agents include lecithin and polyethylene oxide sorbitan (e.g. polysorbate 80). Typical preservatives include methyl paraben and sodium benzoate. Peroral liquid compositions may also contain one or more components such as sweeteners, flavoring agents and colorants disclosed above.

Other compositions useful for attaining systemic delivery include sublingual and buccal dosage forms. Such compositions may comprise one or more of soluble filler substances such as sucrose, sorbitol and mannitol; and binders such as acacia, microcrystalline cellulose, carboxymethyl cellulose and hydroxypropyl methyl cellulose. Glidants, lubricants, sweeteners, colorants, antioxidants and flavoring agents disclosed above may also be included.

Compositions can also be used to deliver the compound to the site where activity is desired; such as eye drops, gels and creams for ocular disorders.

Compositions for use in the methods of this invention may comprise solutions or emulsions, which in some embodiments are aqueous solutions or emulsions comprising a safe and effective amount of a SARM and optionally, other compounds, intended for topical intranasal administration. Such compositions may comprise from about 0.01% to about 10.0% w/v of a subject compound, more preferably from about 0.1% to about 2.0, which may be used for systemic delivery of the compounds by the intranasal route.

The compositions may also comprise preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfote and others; aromatic agents; viscosity adjustors, such as polymers, including cellulose and derivatives thereof; and polyvinyl alcohol and acids and bases to adjust the pH of these aqueous compositions as needed. The compositions may also comprise local anesthetics or other actives. The compositions can be used as sprays, mists, drops, and the like.

Other compositions comprise dry powders. Compositions may be formulated for atomization and inhalation administration. Such compositions may be contained in a container with attached atomizing means. Such compositions may comprise propellants such as chlorofluorocarbons 12/11 and 12/114, or, in another embodiment, other fluorocarbons, nontoxic volatiles; solvents such as water, glycerol and ethanol, these include cosolvents as needed to solvate or suspend the active; stabilizers such as ascorbic acid, sodium metabisulfite; preservatives such as cetylpyridinium chloride and benzalkonium chloride; tonicity adjustors such as sodium chloride; buffers; and flavoring agents such as sodium saccharin.

Other compositions of this invention useful for peroral administration include solids, such as tablets and capsules, and liquids, such as solutions, suspensions and emulsions (for example in soft gelatin capsules). Such compositions can be coated by conventional methods, including, in some embodiments, with pH or time-dependent coatings, such that the subject compound is released in the gastrointestinal tract at various times to extend the desired action. Such dosage forms comprise, but are not limited to, one or more of cellulose acetate phthalate, polyvinylacetate phthalate, hydroxypropyl methyl cellulose phthalate, ethyl cellulose, Eudragit™ coatings, waxes and shellac.

The compounds for use according to the methods of the invention may be administered by ocular, oral, parenteral, including, for example, using formulations suitable as eye drops. For ocular administration, ointments or droppable liquids may be delivered by ocular delivery systems known to the art such as applicators or eye droppers. Such compositions can include mucomimetics such as hyaluronic acid, chondroitin sulfate, hydroxypropyl methylcellulose or polyvinyl alcohol, preservatives such as sorbic acid, EDTA or benzylchromium chloride, and the usual quantities of diluents and/or carriers. See, Remington's Pharmaceutical Sciences, 16th Ed., Mack Publishing, Easton, Pa., 1980, as well as later editions, for information on pharmaceutical compounding.

Numerous additional administration vehicles will be apparent to those of ordinary skill in the art, including without limitation slow release formulations, liposomal formulations and polymeric matrices.

In another embodiment, the composition will comprise a SARM formulated for administration at a dose of approximately 0.1 or 0.5 to 4 mg/kg body weight daily.

Various embodiments of dosage ranges are contemplated by this invention. The dosage may be in the range of 0.1-80 mg/day. In another embodiment, the dosage is in the range of 0.1-50 mg/day. In another embodiment, the dosage is in the range of 0.1-20 mg/day. In another embodiment, the dosage is in the range of 0.1-10 mg/day. In another embodiment, the dosage is in the range of 0.1-5 mg/day. In another embodiment, the dosage is in the range of 0.5-5 mg/day. In another embodiment, the dosage is in the range of 0.5-50 mg/day. In another embodiment, the dosage is be in the range of 5-80 mg/day. In another embodiment, the dosage is in the range of 35-65 mg/day. In another embodiment, the dosage is in the range of 35-65 mg/day. In another embodiment, the dosage is in the range of 20-60 mg/day. In another embodiment, the dosage is in the range of 40-60 mg/day. In another embodiment, the dosage is in a range of 45-60 mg/day. In another embodiment, the dosage is in the range of 40-60 mg/day. In another embodiment, the dosage is in a range of 60-120 mg/day. In another embodiment, the dosage is in the range of 120-240 mg/day. In another embodiment, the dosage is in the range of 40-60 mg/day. In another embodiment, the dosage is in a range of 240-400 mg/day. In another embodiment, the dosage is in a range of 45-60 mg/day. In another embodiment, the dosage is in the range of 15-25 mg/day. In another embodiment, the dosage is in the range of 5-10 mg/day. In another embodiment, the dosage is in the range of 55-65 mg/day. In one embodiment, the dosage is 20 mg/day. In another embodiment, the dosage is 40 mg/day. In another embodiment, the dosage is 60 mg/day.

The pharmaceutical compositions containing the SARM agent can be administered to a subject by any method known to a person skilled in the art, such as parenterally, paracancerally, transmucosally, transdermally, intramuscularly, intravenously, intradermally, subcutaneously, intraperitonealy, intraventricularly, intracranially, intravaginally or intratumorally.

In one embodiment, the pharmaceutical compositions are administered orally, and are thus formulated in a form suitable for oral administration, i.e. as a solid or a liquid preparation. Suitable solid oral formulations include tablets, capsules, pills, granules, pellets and the like. Suitable liquid oral formulations include solutions, suspensions, dispersions, emulsions, oils and the like. In one embodiment of the present invention, the SARM compounds are formulated in a capsule. In accordance with this embodiment, the compositions of the present invention comprise in addition to the SARM active compound and the inert carrier or diluent, a hard gelating capsule.

Further, in another embodiment, the pharmaceutical compositions are administered by intravenous, intraarterial, or intramuscular injection of a liquid preparation. Suitable liquid formulations include solutions, suspensions, dispersions, emulsions, oils and the like. In one embodiment, the pharmaceutical compositions are administered intravenously, and are thus formulated in a form suitable for intravenous administration. In another embodiment, the pharmaceutical compositions are administered intraarterially, and are thus formulated in a form suitable for intraarterial administration. In another embodiment, the pharmaceutical compositions are administered intramuscularly, and are thus formulated in a form suitable for intramuscular administration.

Further, in another embodiment, the pharmaceutical compositions are administered topically to body surfaces, and are thus formulated in a form suitable for topical administration. Suitable topical formulations include gels, ointments, creams, lotions, drops and the like. For topical administration, the SARM agents or their physiologically tolerated derivatives such as salts, esters, *N*-oxides, and the like are prepared and applied as solutions, suspensions, or emulsions in a physiologically acceptable diluent with or without a pharmaceutical carrier.

Further, in another embodiment, the pharmaceutical compositions are administered as a suppository, for example a rectal suppository or a urethral suppository. Further, in another embodiment, the pharmaceutical compositions are administered by subcutaneous implantation of a pellet. In a further embodiment, the pellet provides for controlled release of SARM agent over a period of time.

In another embodiment, the active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

In addition, the compositions may further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hyroxypropylmethyl cellulose), viscosity increasing agents(e.g. carbomer, colloidal silicon dioxide, ethyl cellulose, guar gum), sweetners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants.

In one embodiment, the pharmaceutical compositions provided herein are controlled release compositions, i.e. compositions in which the SARM compound is released over a period of time after administration. Controlled or sustained release compositions include formulation in lipophilic depots (e.g. fatty acids, waxes, oils). In another embodiment, the composition is an immediate release composition, i.e. a composition in which all of the SARM compound is released immediately after administration.

In another embodiment, the pharmaceutical composition delivered in a controlled release system may be formulated for intravenous infusion, implantable osmotic pump, transdermal patch, liposomes, or other modes of administration. In one embodiment, a pump may be used (see Langer, supra; Sefton, CRC Crit. Ref. Biomed. Eng. 14:201 (1987); Buchwald et al., Surgery 88:507 (1980); Saudek et al., N. Engl. J. Med. 321:574

(1989). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

The compositions may also include incorporation of the active material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts.) Such compositions will influence the physical state, solubility, stability, rate of *in vivo* release, and rate of *in vivo* clearance.

Also comprehended by the invention are particulate compositions coated with polymers (e.g. poloxamers or poloxamines) and the compound coupled to antibodies directed against tissue-specific receptors, ligands or antigens or coupled to ligands of tissue-specific receptors.

Also comprehended by the invention are compounds modified by the covalent attachment of watersoluble polymers such as polyethylene glycol, copolymers of polyethylene glycol and polypropylene glycol, carboxymethyl cellulose, dextran, polyvinyl alcohol, polyvinylpyrrolidone or polyproline. The modified compounds are known to exhibit substantially longer half-lives in blood following intravenous injection than do the corresponding unmodified compounds (Abuchowski et al., 1981; Newmark et al., 1982; and Katre et al., 1987). Such modifications may also increase the compound's solubility in aqueous solution, eliminate aggregation, enhance the physical and chemical stability of the compound, and greatly reduce the immunogenicity and reactivity of the compound. As a result, the desired *in vivo* biological activity may be achieved by the administration of such polymer-compound abducts less frequently or in lower doses than with the unmodified compound.

The preparation of pharmaceutical compositions which contain an active component is well understood in the art, for example by mixing, granulating, or tablet-forming processes. The active therapeutic ingredient is often mixed with excipients which are pharmaceutically acceptable and compatible with the active ingredient. For oral administration, the SARM agents or their physiologically tolerated derivatives such as salts, esters, *N-*oxides, and the like are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions. For parenteral administration, the SARM agents or their physiologically tolerated derivatives such as salts, esters, *N*-oxides, and the like are converted into a solution, suspension, or emulsion, if desired with the substances customary and suitable for this purpose, for example, solubilizers or other.

An active component can be formulated into the composition as neutralized pharmaceutically acceptable salt forms. Pharmaceutically acceptable salts include the acid addition salts (formed with the free amino groups of the polypeptide or antibody molecule), which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

For use in medicine, the salts of the SARM will be pharmaceutically acceptable salts. Other salts may, however, be useful in the preparation of the compounds according to the invention or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic: acid, oxalic acid, citric acid, tartaric acid, carbonic acid or phosphoric acid.

### SARM characteristics

SARMs, which are useful in preventing and treating the diseases and/or disorders and/or conditions as described herein, may be classified, in some embodiments, as androgen receptor agonists (AR agonists), partial agonists or androgen receptor antagonists (AR antagonists).

A receptor agonist is a substance which binds receptors and activates them. A receptor partial agonist is a substance which binds receptor and partially activates them. A receptor antagonist is a substance which binds receptors and inactivates them. The SARM compounds for use in the methods of the present invention have a tissue-selective effect, wherein one agent may be an agonist, partial agonist and/or antagonist, depending on the tissue wherein the SARM is found. For example, the SARM compound may stimulate muscle tissue, thereby exhibit anabolic activity, or prevent signaling through the androgen receptor in the prostate, thereby inhbiting anti-androgenic activity. In one embodiment, the SARMs which are useful in the methods of this invention are agonsits. In another embodiment, the SARMs are AR antagonists, and are, therefore, useful in binding to and inactivating the AR. Assays to determine whether the compounds of the present invention are AR agonists or antagonists are well known to a person skilled in the art. For example, AR agonistic activity can be determined by monitoring the ability of the SARM compounds to maintain and/or stimulate the growth of AR containing tissue such as prostate and seminal vesicles, as measured by weight. AR antagonistic activity can be determined by monitoring the ability of the SARM compounds inhibit the growth of AR containing tissue.

In another embodiment, the SARM compounds of the present invention can be classified as partial AR agonist/antagonists. In one embodiment, SARMs are AR agonists in particular tissues, promoting transcription of AR-responsive genes (e.g. muscle anabolic effect), and concurrently, in other tissues, these compounds serve as competitive inhibitors of testosterone/DHT binding to the AR, preventing native androgen binding, thereby being anti-androgenic.

The SARM compounds for use in the methods of the present invention bind either reversibly or irreversibly to the androgen receptor. In one embodiment, the SARM compounds bind reversibly to the androgen receptor. In another embodiment, the SARM compounds bind irreversibly to the androgen receptor. The compounds of the present invention may contain a functional group (affinity label) that allows alkylation of the androgen receptor (i.e. covalent bond formation). Thus, in this case, the compounds bind irreversibly to the receptor and, accordingly, cannot be displaced by a steroid, such as the endogenous ligands DHT and testosterone.

In one embodiment, the methods of the present invention comprise administering a SARM compound as the sole active ingredient. However, also encompassed within the scope of the present invention are methods of treatment, which comprise administering the SARM compounds in combination with one or more therapeutic agents, as described herein. These agents may, in some embodiments, comprise LHRH analogs, reversible antiandrogens, antiestrogens, selective estrogen receptor modulators (SERMS), anticancer drugs, 5-alpha reductase inhibitors, aromatase inhibitors, progestins, other selective androgen receptor modulators (SARMS), testosterone, anabolic steroids, growth hormones or agents acting through other nuclear hormone receptors, or any combination thereof.

Thus, in one embodiment, the present invention provides for the use of compositions comprising a selective androgen receptor modulator compound, in combination with an LHRH analog. In another embodiment, the present invention provides for the use of compositions comprising a selective androgen receptor modulator compound, in combination with a reversible antiandrogen. In another embodiment, the present invention provides for the use of compositions comprising a selective androgen receptor modulator compound, in combination with an antiestrogen. In another embodiment, the present invention provides for the use of compositions comprising a selective androgen receptor modulator compound, in combination with a SERM. In another embodiment, the present invention provides for the use of compositions comprising a selective androgen receptor modulator compound, in combination with an anticancer drug. In another embodiment, the present invention provides for the use of compositions comprising a selective androgen receptor modulator compound, in combination with a 5-alpha reductase inhibitor. In another embodiment, the present invention provides for the use of compositions comprising a selective androgen receptor modulator compound, in combination with an aromatase inhibitor. In another embodiment, the present invention provides for the use of compositions comprising a selective androgen receptor modulator compound, in combination with a progestin. In another embodiment, the present invention provides for the use of compositions comprising a selective androgen receptor modulator compound, in combination with another SARM. In another embodiment, the present invention provides for the use of compositions comprising a selective androgen receptor modulator compound, in combination with testosterone. In another embodiment, the present invention provides for the use of compositions comprising a selective androgen receptor modulator compound, in combination with an anabolic steroid. In another embodiment, the present invention provides for the use of compositions comprising a selective androgen receptor modulator compound, in combination with a growth hormone. In another embodiment, the present invention provides for the use of compositions comprising a selective androgen receptor modulator compound, in combination with an agent acting through other nuclear hormone receptors.

### Activity of Selective Androgen Modulator Compounds

### Kidney,disease and related conditions

In one embodiment, the present invention provides a method of treating a subject suffering from, or predisposed to a kidney disease or disorder, comprising the step of administering to the subject a selective androgen receptor modulator (SARM) compound and/or its analog, derivative, isomer, metabolite, pharmaceutically acceptable salt, pharmaceutical product, hydrate, *N*-oxide, prodrug, polymorph, impurity, crystal or any combination thereof.

In another embodiment, the present invention provides a method of preventing a kidney disease or disorder in a subject, comprising the step of administering to the subject a selective androgen receptor modulator (SARM) compound and/or its analog, derivative, isomer, metabolite, pharmaceutically acceptable salt, pharmaceutical product, hydrate, *N*-oxide, prodrug, polymorph, impurity, crystal or any combination thereof.

In another embodiment, the present invention provides a method of treating, preventing, suppressing, inhibiting or reducing the incidence of symptoms associated with a kidney disease or disorder in a subject, comprising the step of administering to the subject a selective androgen receptor modulator (SARM) compound and/or its analog, derivative, isomer, metabolite, pharmaceutically acceptable salt, pharmaceutical product, hydrate, *N*-oxide, prodrug, polymorph, impurity, crystal or any combination thereof.

In one embodiment, the kidney disease or disorder is acute, or in another embodiment, chronic. In one embodiment, clinical indications of a kidney disease or disorder, wherein the treatment may be useful include urinary casts, measured GFR, or other markers of renal function.

In one embodiment, the methods of this invention are useful in subjects predisposed to kidney diseases or disorders. In one embodiment, the phrase "predisposed to a kidney disease or disorder" with respect to a subject is synonymous with the phrase "subject at risk", and includes a subject at risk of acute or chronic renal failure, or at risk of the need for renal replacement therapy, if the subject is reasonably expected to suffer a progressive loss of renal function associated with progressive loss of functioning nephron units. Whether a particular subject is at risk is a determination which may routinely be made by one of ordinary skill in the relevant medical or veterinary art.

Subjects at risk of chronic renal failure, for example, or at risk of the need for renal replacement therapy, include but are not limited to the following: subjects which may be regarded as afflicted with chronic renal failure, end-stage renal disease, chronic diabetic nephropathy, hypertensive nephrosclerosis, chronic glomerulonephritis, hereditary nephritis, and/or renal dysplasia; subjects having a biopsy indicating glomerular hypertrophy, tubular hypertrophy, chronic glomerulosclerosis, and/or chronic tubulointerstitial sclerosis; subjects having an ultrasound, MRI, CAT scan, or other non-invasive examination indicating renal fibrosis; subjects having an unusual number of broad casts present in urinary sediment; subjects having a GFR which is chronically less than about 50%, and more particularly less than about 40%, 30% or 20%, of the expected GFR for the subject; human male subjects weighing at least about 50 kg and having a GFR which is chronically less than about 50 ml/min, or less than about 40 ml/min, 30 ml/min or 20 ml/min; human female subjects weighing at least about 40 kg and having a GFR which is chronically less than about 40 ml/min, or less than about 30 ml/min, 20 ml/min or 10 ml/min; subjects possessing a number of functional nephron units which is less than about 50%, or less than about 40%, 30% or 20%, of the number of functional nephron units possessed by a healthy but otherwise similar subject; subjects which have a single kidney; or subjects which have received a kidney transplant.

The methods of the present invention may be utilized for any mammalian subject needing the indicated treatment. Mammalian subjects which may be treated according to the methods of the invention include, but are not limited to, human subjects or patients.

In addition, however, the invention may be employed in the treatment of domesticated mammals which are maintained as human companions (e.g., dogs, cats, horses), which have significant commercial value (e.g., dairy cows. beef cattle, sporting animals), which have significant scientific value (e.g., captive or free specimens of endangered species), or which otherwise have value.

One of ordinary skill in the medical or veterinary arts is trained to recognize subjects in need for the treatment/preventive methods of this invention. In particular, clinical and non-clinical trials, as well as accumulated experience, relating to the presently disclosed and other methods of treatment, are expected to inform the skilled practitioner in deciding whether a given subject is at risk of a condition, disease or disorder for treatment by the methods of this invention, for example, at risk of chronic renal disease, such as end stage renal disease, or at risk of needing renal replacement therapy, etc., and whether any particular treatment is best suited to the subject's needs, including treatment according to the present invention.

In one embodiment, a mammalian subject may be regarded as being at risk of chronic renal failure, or at risk of needing renal replacement therapy, if that subject has already been diagnosed as afflicted with, or would be regarded as being afflicted with, a condition which typically leads to progressive loss of renal function associated with progressive loss of functioning nephron units. Such conditions include, but are not limited to, chronic renal failure, end-stage renal disease, chronic diabetic nephropathy, hypertensive nephrosclerosis, chronic glomerulonephritis, hereditary nephritis, renal dysplasia and the like. These, and other diseases and conditions known in the art, typically lead to a progressive loss of functioning nephrons and to the onset of chronic renal failure.

Frequently, one of skill in the medical or veterinary arts may base a prognosis, diagnosis or treatment decision upon an examination of a renal biopsy sample. Such biopsies provide a wealth of information useful in diagnosing disorders of the kidney but, due to the invasiveness of the procedure, and the additional trauma to a presumably unhealthy kidney, may not be appropriate for all subjects. Nonetheless, subjects at risk of chronic renal failure, or at risk of needing renal replacement therapy, may be recognized by histological indications from renal biopsies including, but not limited to, glomerular hypertrophy, tubular hypertrophy, glomerulosclerosis, tubulointerstitial sclerosis, and the like.

Less invasive techniques for assessing kidney morphology include MRI, CAT and ultrasound scans. Scanning techniques are also available which employ contrasting or imaging agents (e.g., radioactive dyes) but, it should be noted, some of these are particularly toxic to renal tissues and structures and, therefore, their use may be ill-advised in subjects at risk of chronic renal failure. Such non-invasive scanning techniques may be employed to detect conditions such as renal fibrosis or sclerosis, focal renal necrosis, renal cysts, and renal gross hypertrophy which will place a subject at risk of chronic renal failure, or at risk of needing renal replacement therapy.

Prognosis, diagnosis and/or treatment decisions may be based, in some embodiments, upon clinical indications of renal function. One such indication is the presence in urinary sediment of an unusual number of "broad" or "renal failure" casts, which is indicative of tubular hypertrophy and suggests the compensatory renal hypertrophy which typifies chronic renal failure. Another indication of renal function is the glomerular flow rate (GFR), which can be measured directly by quantifying the rate of clearance of particular markers, or which may be inferred from indirect measurements.

It is important to note that the methods of treatment of the present invention need not be restricted to subjects presenting with any particular measures of GFR, or other particular marker of renal function.

In one embodiment, subjects with kidney disease, in particular male subjects with end-stage renal disease (ESRD) suffer from hypogonadism, with some having concomitant moderate to severe protein-energy malnutrition (PEM), which leads to higher required doses of EPO, lower QOL scores, and higher mortality. Many have other symptoms associated with hypogonadism, including fatigue, lack of apetite, muscle weakness, etc. In some embodiments, the treatment methods of this invention are useful in treating symptoms associated with hypogonadism, brought about in the subject by the kidney disease or disorder.

In one embodiment, the subject for whom treatment is sought via the methods of this invention is one with diabetic nephropathy. Diabetic nephropathy is a complication of diabetes that evolves early, typically before clinical diagnosis of diabetes is made. The earliest clinical evidence of nephropathy is the appearance of low but abnormal levels (>30 mg/day or 20 µg/min) of albumin in the urine (microalbuminuria), followed by albuminuria (>300 mg/24 h or 200 µg/min) that develops over a period of 10-15 years. In patients with type 1 diabetes, diabetic hypertension typically becomes manifest early on, by the time that patients develop microalbuminuria. Once overt nephropathy occurs, the glomerular filtration rate (GFR) falls over a course of times, which may be several years, resulting in End Stage Renal Disease (ESRD) in diabetic individuals.

In one embodiment, the terms "treatment" or "treating" refer to preventative as well as disorder remitative treatment. In one embodiment, the terms "reducing", "suppressing" or "inhibiting" refer to lessening, delaying or decreasing, and may refer to symptoms, markers, associated with the disease or disorder, as well as the underlying cause of the disease or disorder.

In one embodiment, the term "administering" refers to bringing a subject in contact with a SARM compound of the present invention, which may be accomplished *in vitro,* i.e. in a test tube, or *in vivo,* i.e. in cells or tissues of living organisms, for example humans, or ex-vivo, in terms of implanting cells which have been treated, prior to their implantation, In one embodiment, the present invention encompasses administering the compounds of the present invention to a subject, through any route, as will be appreciated by one skilled in the art.

In one embodiment of this invention, treatment methods for preventing, ameliorating, inhibiting, treating, or delaying kidney diseases or disorders may comprise administration of other compounds in addition to a SARM. In one embodiment, the additional compound may be related to other causative factors in the development, or in another embodiment, predisposition to a kidney disease or disorder. For example, in one embodiment, treatment of diabetics at risk for, or in one embodiment, suffering from a kidney disease or disorder, or a precursor thereto, may be treated with an effective amount of a thiazolidinedione or "glitazone" diabetes drug, such as Troglitazone, Rosiglitazone, and Pioglitazone, in addition to SARM treatment.

In another embodiment, other anti-diabetic agents may be administered in combination with a SARM. Such anti-diabetic agents may include biguanides (e.g., metformin), glucosidase inhibitors (e.g., acarbose), insulins (including insulin secretagogues or insulin sensitizers), meglitinides (e.g., repaglinide), sulfonylureas (e.g., glimepiride, glyburide and glipizide), biguanide/glyburide combinations (e.g., Glucovance®), thiazolidinediones (e.g., troglitazone, rosiglitazone and pioglitazone), PPAR-alpha agonists, PPAR-gamma agonists, PPAR alpha/gamma dual agonists, SGLT2 inhibitors, glycogen phosphorylase inhibitors, inhibitors of fatty acid binding protein (aP2) such as those disclosed in U.S. Ser. No. 09/519,079 filed Mar. 6, 2000, glucagon-like peptide-1 (GLP-1), and dipeptidyl peptidase IV (DPP4) inhibitors such as those disclosed in WO 0168603.

The combined therapy may be co-administered in a single composition to a subject, in one embodiment, or in another embodiment, the compounds may be administered separately, with time, route, composition, etc., being varied for administration of each respective compound, for any of the treatment methods of this invention.

Such other therapeutic agents may include, for example, one or more inhibitors of ileal bile transporter activity ("IBAT inhibitors"), inhibitors of cholesterol ester transfer protein activity ("CETP inhibitors"), fibrates, digoxin, calcium channel blockers, endothelin antagonists, inhibitors of microsomal triglyceride transfer protein, cholesterol absorption antagonists, phytosterols, bile acid sequestrants, vasodilators, adrenergic blockers, adrenergic stimulants, and/or inhibitors of HMG-CoA reductase activity. Such other therapeutic agents may also comprise, for example, one or more conventional antiinflammatories, such as steroids, cyclooxygenase-2 inhibitors, disease-modifying anti-rheumatic drugs ("DMARDs"), immunosuppressive agents, non-steroidal anti-inflammatory drugs ("NSAIDs"), 5-lipoxygenase inhibitors, LTB4 antagonists, and LTA4 hydrolase inhibitors.

In one embodiment, erythropoietin is administered with the SARMs for the methods of this invention, which, in one embodiment, is useful in treating subjects with, or predisposed to a kidney disease or disorder.

In one embodiment, the erythropoietin used according to the methods of this invention is obtained by natural sources (e.g., urinary erythropoietin; See U.S. Pat. 3,865,801), or is a recombinantly produced protein and analogs thereof, for example, as described in U.S. Pat. Nos. 5,441,868, 5,547,933, 5,618,698 and 5,621,080 as well as human erythropoietin analogs with increased glycosylation and/or changes in the amino acid sequence as those described in European Patent Publication No. EP 668351 and the hyperglycosylated analogs having 1-14 sialic acid groups and changes in the amino acid sequence described in PCT Publication No. WO 91/05867.

Erythropoietin-like polypeptides are also encompassed by the present invention, including, e.g., darbepoietin (from Amgen; also known as Aranesp and novel erthyropoiesis stimulating protein (NESP)). Administration of darbepoietin for use in the present invention includes subcutaneous or intravenous administration at about 0.5 micrograms/kg once a week.

Erythropoietin serum concentration is normally within the range of 5-50 mU/ml, however, in patients suffering from chronic renal failure or other conditions involving anemia, erythropoietin is much less, and treatment comprises attempts to raise and maintain the blood concentration at about 1-100 mU/ml, in one embodiment. This plasma concentration may be achieved through administration of one to several doses a day.

Hypertension is another comorbid factor for renal disease. In some embodiments, treatment of renal disease may comprise concomitant treatment with a SARM and an agent which treats hypertension.

Examples of suitable anti-hypertensive agents for use in combination with the SARMs include beta adrenergic blockers, calcium channel blockers (L-type and T-type; e.g. diltiazem, verapamil, nifedipine, amlodipine and mybefradil), diuretics (e.g., chlorothiazide, hydrochlorothiazide, flumethiazide, hydroflumethiazide, bendroflumethiazide, methylchlorothiazide, trichloromethiazide, polythiazide, benzthiazide, ethacrynic acid tricrynafen, chlorthalidone, furosemide, musolimine, bumetanide, triamtrenene, amiloride, spironolactone), renin inhibitors, ACE inhibitors (e.g., captopril, zofenopril, fosinopril, enalapril, ceranopril, cilazopril, delapril, pentopril, quinapril, ramipril, lisinopril), AT-1 receptor antagonists (e.g., losartan, irbesartan, valsartan), ET receptor antagonists (e.g., sitaxsentan, atrsentan and compounds disclosed in U.S. Pat. Nos. 5,612,359 and 6,043,265), Dual ET/AII antagonist (e.g., compounds disclosed in WO 00/01389), neutral endopeptidase (NEP) inhibitors, vasopepsidase inhibitors (dual NEP-ACE inhibitors) (e.g., omapatrilat and gemopatrilat), and nitrates.

Other compounding factors are hypercholesterolemia, atherosclerosis, thrombosis and others, as will be appreciated by the skilled artisan. In one embodiment, the methods of this invention comprise administration of at least one SARM in combination with agents that address these factors, as well, alone or in combination with other agents as herein described.

Examples of suitable anti-platelet agents for use in combination with a SARM include GPIIb/IIIa blockers (e.g., abciximab, eptifibatide, tirofiban), P2Y12 antagonists (e.g., clopidogrel, ticlopidine, CS-747), thromboxane receptor antagonists (e.g., ifetroban), aspirin, and PDE-III inhibitors (e.g., dipyridamole) with or without aspirin. Examples of suitable cardiac glycosides for use in combination a SARM include digitalis and ouabain. Examples of suitable cholesterol/lipid lowering agents for use in combination with a SARM include HMG-CoA reductase inhibitors (e.g., pravastatin, lovastatin, atorvastatin, simvastatin, NK-104 (a.k.a. itavastatin, or nisvastatin or nisbastatin) and ZD-4522 (a.k.a. rosuvastatin, or atavastatin or visastatin)), squalene synthetase inhibitors, fibrates, bile acid sequestrants, ACAT inhibitors, MTP inhibitors, lipooxygenase inhibitors, cholesterol absorption inhibitors, and cholesterol ester transfer protein inhibitors (e.g., CP-529414).

Animal models may be used to evaluate regimens for the treatment methods proposed in the present invention. For example, rats having undergone renal mass reduction (RMR) by nephrectomy may be assessed for the degree of glomerulosclerosis, mesangial expansion and proliferation and tubulo-interstitial changes, as a function of the treatment regimen, in terms of the compound, dose, or combinations used.

### Wasting or Cachexia and SARM treatment

In another embodiment, the present invention provides a method of treating, preventing, inhibiting, reducing or suppressing, or ameliorating symptoms associated with cachexia or involuntary weight loss in a subject, comprising the step of administering to the subject a selective androgen receptor modulator (SARM) compound and/or its analog, derivative, isomer, metabolite, pharmaceutically acceptable salt, pharmaceutical product, hydrate, N-oxide, prodrug, polymorph, impurity, crystal or any combination thereof.

In another embodiment, the present invention provides a method of treating, preventing, inhibiting, reducing or suppressing muscle wasting in a subject suffering from a kidney disease or disorder, comprising the step of administering to the subject a selective androgen receptor modulator (SARM) compound and/or its analog, derivative, isomer, metabolite, pharmaceutically acceptable salt, pharmaceutical product, hydrate, *N-*oxide, prodrug, polymorph, impurity, crystal or any combination thereof.

In another embodiment, the present invention provides a method of treating, preventing, inhibiting, reducing or suppressing protein catabolism in a subject suffering from a kidney disease or disorder, comprising the step of administering to the subject a selective androgen receptor modulator (SARM) compound and/or its analog, derivative, isomer, metabolite, pharmaceutically acceptable salt, pharmaceutical product, hydrate, *N-*oxide, prodrug, polymorph, impurity, crystal or any combination thereof.

As contemplated herein, the SARM compounds of the present invention as useful in treating, preventing, suppressing, inhibiting or reducing the incidence of muscle wasting, associated with the conditions for treatment as described herein.

An intact androgen receptor (AR) signaling pathway is crucial, *inter-alia,* for appropriate development of skeletal muscles. Furthermore, an intact AR-signalling pathway increases lean muscle mass, muscle strength and muscle protein synthesis, whose increase is useful in treating symptoms associated with the conditions, diseases or disorders described herein, subject to the methods of this invention.

A wasting condition or disorder is defined herein as a condition or disorder that is characterized, at least in part, by an abnormal, progressive loss of body, organ or tissue mass. A wasting condition can occur as a result of a pathology such as, for example, cancer, or it can be due to a physiologic or metabolic state, such as disuse deconditioning that can occur, for example, due to prolonged bed rest or when a limb is immobilized, such as in a cast, or with the occurrence of multiple wounds, including, for example, amputation, as occurs in diabetics, and other conditions, as will be appreciated by one skilled in the art. A wasting condition can also be age associated. The loss of body mass that occurs during a wasting condition can be characterized by a loss of total body weight, or a loss of organ weight such as a loss of bone or muscle mass due to a decrease in tissue protein.

In one embodiment, the terms "muscle wasting" or "muscular wasting", refer to the progressive loss of muscle mass and/or to the progressive weakening and degeneration of muscles, including the skeletal or voluntary muscles which control movement, cardiac muscles which control the heart, and smooth muscles. In one embodiment, the muscle wasting condition or disorder is a chronic muscle wasting condition or disorder. "Chronic muscle wasting" is defined herein as the chronic (i.e. persisting over a long period of time) progressive loss of muscle mass and/or to the chronic progressive weakening and degeneration of muscle.

The loss of muscle mass that occurs during muscle wasting can be characterized by a muscle protein breakdown or degradation, by muscle protein catabolism. Protein catabolism occurs because of an unusually high rate of protein degradation, an unusually low rate of protein synthesis, or a combination of both. Protein catabolism or depletion, whether caused by a high degree of protein degradation or a low degree of protein synthesis, leads to a decrease in muscle mass and to muscle wasting. The term "catabolism" has its commonly known meaning in the art, specifically an energy burning form of metabolism.

Muscle wasting can occur as a result of a pathology, disease, condition or disorder, including disorders for treatment via the methods of this invention, such as, for example, end stage Renal failure.

Cachexia is weakness and a loss of weight caused by a disease or as a side effect of illness. Long term hospitalization due to illness or injury, or disuse deconditioning that occurs, for example, when a limb is immobilized, can also lead to muscle wasting. Studies have shown that in patients suffering injuries, chronic illnesses, bums, trauma or cancer, who are hospitalized for long periods of time, there is a long-lasting unilateral muscle wasting, with a consequent decrease in body mass. Nervous system injury, for example, spinal cord injury, as described further herein, may be a contributory factor, as well. Any of these conditions, whether the primary or secondary condition sought to be treated may be treated via the administration of at least one SARM compound, or in another embodiment, a combination of SARMs, or in another embodiment, SARM or SARMs in combination with other agents, to ameliorate, prevent, reduce or treat the conditions and/or symptoms thereof, as herein described, and represent embodiments of what is to be considered the methods of this invention.

The compounds may be combined with growth promoting agents, such as, but not limited to, TRH, diethylstilbesterol, theophylline, enkephalins, E series prostaglandins, compounds disclosed in U.S. Pat. No. 3,239,345, e.g., zeranol, and compounds disclosed in U.S. Pat. No. 4,036,979, e.g., sulbenox or peptides disclosed in U.S. Pat. No. 4,411,890.

In other embodiments, the methods may he effected via coadminstration of growth hormone secretagogues such as GHRP-6, GHRP-1 (as described in U.S. Pat. No. 4,411,890 and publications WO 89/07110 and WO 89/07111), GHRP-2 (as described in WO 93/04081), NN703 (Novo Nordisk), LY444711 1 (Lilly), MK-677 (Merck), CP424391 (Pfizer) and B-HT920, or, in other embodiments, with growth hormone releasing factor and its analogs or growth hormone and its analogs or somatomedins including IGF-1 and IGF-2, or with alpha-adrenergic agonists, such as clonidine or serotinin 5-HTD agonists, such as sumatriptan, or agents which inhibit somatostatin or its release, such as physostigmine and pyridostigmine. In another embodiment, the methods may be effected via coadminstration of parathyroid hormone, PTH(1-34) or bisphosphonates, such as MK-217 (alendronate).

In another embodiment, the methods may be effected via coadminstration of estrogen, testosterone, a selective estrogen receptor modulator, such as tamoxifen or raloxifene, or other androgen receptor modulators, such as those disclosed in Edwards, J. P. et. al., Bio. Med. Chem. Let., 9,1003-1008 (1999) and Hamann, L. G. et. al., J. Med. Chem., 42, 210-212 (1999).

In another embodiment, the methods may be effected via coadminstration of progesterone receptor agonists ("PRA"), such as levonorgestrel, medroxyprogesterone acetate (MPA).

In another embodiment, the methods may be effected via coadminstration of nuclear hormone receptors or other suitable therapeutic agents useful in the treatment of the aforementioned disorders including: anti-diabetic agents; anti-osteoporosis agents; anti-obesity agents; anti-inflammatory agents; anti-anxiety agents; anti-depressants; anti-hypertensive agents; anti-platelet agents; anti-thrombotic and thrombolytic agents; cardiac glycosides; cholesterol/lipid lowering agents; mineralocorticoid receptor antagonists; phospodiesterase inhibitors; protein tyrosine kinase inhibitors; thyroid mimetics (including thyroid receptor agonists); anabolic agents; HIV or AIDS therapies; therapies useful in the treatment of Alzheimer's disease and other cognitive disorders; therapies useful in the treatment of sleeping disorders; anti-proliferative agents or anti-tumor agents, or any combination thereof.

Examples of suitable mineralocorticoid receptor antagonists for use in combination with a SARM, according to the methods of this invention include spironolactone and eplerinone.

Examples of suitable phospodiesterase (PDE) inhibitors for use in combination with a SARM, according to the methods of this invention include PDE-3 inhibitors such as cilostazol, and phosphodiesterase-5 inhibitors (PDE-5 inhibitors) such as sildenafil.

Examples of suitable thyroid mimetics for use in combination with a SARM, according to the methods of this invention include thyrotropin, polythyroid, KB-130015, and dronedarone.

Examples of suitable anabolic agents for use in combination with a SARM, according to the methods of this invention include testosterone, TRH diethylstilbesterol, estrogens, β-agonists, theophylline, anabolic steroids, dehydroepiandrosterone, enkephalins, E-series prostagladins, retinoic acid and compounds as disclosed in U.S. Pat. No. 3,239,345, e.g., Zeranol®; U.S. Pat. No. 4,036,979, e.g., Sulbenox® or peptides as disclosed in U.S. Pat. No. 4,411,890.

In some embodiments, the methods of this invention may further comprise the administration of nutritional supplements to the subject, such as those described in U.S. Pat. No. 5,179,080, which, in other embodiments are in combination with whey protein or casein, amino acids (such as leucine, branched amino acids and hydroxymethylbutyrate), triglycerides, vitamins (e.g., A, B6, B 12, folate, C, D and E), minerals (e.g., selenium, magnesium, zinc, chromium, calcium and potassium), camitine, lipoic acid, creatinine, B-hyroxy-B-methylbutyriate (Juven) and coenzyme Q.

In some embodiments, the methods of this invention may further comprise the administration of antiresorptive agents, hormone replacement therapies, vitamin D analogues, elemental calcium and calcium supplements, cathepsin K inhibitors, MMP inhibitors, vitronectin receptor antagonists, Src SH2 antagonists, vacular-H+-A.TPase inhibitors, ipriflavone, fluoride, Tibolone, prostanoids, 17-beta hydroxysteroid dehydrogenase inhibitors and Src kinase inhibitors.

### Injury to the Nervous System:

Injuries or damage to the Central Nervous System (CNS) are, in some embodiments, associated with muscle wasting disorders. Injuries or damage to the CNS can be, for example, caused by diseases, trauma or chemicals. Examples are central nerve injury or damage, peripheral nerve injury or damage and spinal cord injury or damage.

Studies involving patients with spinal cord injuries (SCI) have shown that central neurotransmitters may be altered after SCI causing hypothalamus-pituitary-adrenal axis dysfunction, whose disruption led to a significant decrease in testosterone and other hormone levels. SCI or other acute illness or trauma characteristically includes heightened catabolism in conjunction with the lowered anabolic activity resulting in a condition that is prone to loss of lean body tissue, which is often accompanied by disturbed nutrient utilization. The effects of the loss of lean body mass include the development of wounds and impaired healing mechanisms, further compounding the problem. Because of poor nutrition and protein combined with immobilization, patients with spinal cord injury are at high risk for bed sores.

In one embodiment, this invention provides a method of treating a subject suffering from a spinal cord injury, comprising administering a SARM and/or its analog, derivative, isomer, metabolite, pharmaceutically acceptable salt, pharmaceutical product, hydrate, *N*-oxide, impurity, prodrug, polymorph, crystal, or any combination thereof to the subject.

In one embodiment, methods of treating a subject with a spinal cord injury encompass treating any secondary conditions in the subject, which arise due to the subject having a spinal cord injury, some of which are described herein, for example, sores, hypogonadism, cachexia, etc.

In one embodiment, a wide variety of injuries of the CNS may be treated by the methods of the present invention. CNS injury may refer, in one embodiment, to a breakdown of the membrane of a nerve cell, or, in another embodiment, to the inability of the nerve to produce and propagate nerve impulses, or in another embodiment, to the death of the cell. An injury includes damage that directly or indirectly affects the normal functioning of the CNS. The injury may be a structural, physical, or mechanical impairment and may be caused by physical impact, as in the case of a crushing, compression, or stretching of nerve fibers. Alternatively, the cell membrane may be destroyed by or degraded by an illness, a chemical imbalance, or a physiological malfunction such as anoxia (e.g., stroke), aneurysm, or reperfusion. A CNS injury includes, for example and without limitation, damage to retinal ganglion cells, a traumatic brain injury, a stroke-related injury, a cerebral aneurism-related injury, a spinal cord injury, including monoplegia, diplegia, paraplegia, hemiplegia and quadriplegia, a neuroproliferative disorder, or neuropathic pain syndrome.

With injury to the spinal cord of a mammal, connections between nerves in the spinal cord are broken. Such injuries block the flow of nerve impulses for the nerve tracts affected by the injury, with a resulting impairment to both sensory and motor function. Injuries to the spinal cord may arise from compression or other contusion of the spinal cord, or a crushing or severing of the spinal cord. A severing of the spinal cord, also referred to herein as a "transection," may be a complete severing or, may be an incomplete severing of the spinal cord.

The methods of the present invention may be used to treat both acute and chronic injuries of the CNS, including but not limited to acute and chronic spinal cord injuries.

In some embodiments, the methods of treating a subject suffering form a CNS injury or, in other embodiments, spinal cord injury, may be accompanied by treatment of the subject with electrical stimulation of the injured site and the administration of a purine nucleoside, or analog thereof, for example as described in United States Patent Application Publication Number 20040214790A1.

In some embodiments, combined use of a SARM with a treatment of Alzheimer's disease or other cognitive disorders may be desired, and may comprise donepezil, tacrine, revastigmine, 5HT6, gamma secretase inhibitors, beta secretase inhibitors, SK channel blockers, Maxi-K blockers, and KCNQs blockers, melatonin analogs, melatonin receptor antagonists, ML1B agonists, or GABA/NMDA receptor antagonists.

### Burn and Wound Healing

In one embodiment, this invention provides a method of treating a subject suffering from a wound, or reducing the incidence of, or mitigating the severity of, or enhancing or hastening healing of a wound in a subject, the method comprising the step of administering a SARM to said subject.

In one embodiment, this invention provides a method of treating a subject suffering from a burn, or reducing the incidence of, or mitigating the severity of, or enhancing or hastening healing of a burn in a subject, the method comprising the step of administering a SARM to said subject.

Wounds and/or ulcers are normally found protruding from the skin or on a mucosal surface or as a result of an infarction in an organ. A wound may be a result of a soft tissue defect or a lesion or of an underlying condition. In one embodiment, the term "wound" denotes a bodily injury with disruption of the normal integrity of tissue structures. The term is also intended to encompass the terms "sore", "lesion", "necrosis" and "ulcer". In one embodiment, the term "sore" refers to any lesion of the skin or mucous membranes and the term "ulcer" refers to a local defect, or excavation, of the surface of an organ or tissue, which is produced by the sloughing of necrotic tissue. Lesion generally relates to any tissue defect. Necrosis is related to dead tissue resulting from infection, injury, inflammation or infarctions. All of these are encompassed by the term "wound", which denotes any wound at any particular stage in the healing process including the stage before any healing has initiated or even before a specific wound like a surgical incision is made (prophylactic treatment).

Examples of wounds which can be prevented and/or treated in accordance with the present invention are, e.g., aseptic wounds, contused wounds, incised wounds, lacerated wounds, non-penetrating wounds (i.e. wounds in which there is no disruption of the skin but there is injury to underlying structures), open wounds, penetrating wounds, perforating wounds, puncture wounds, septic wounds, subcutaneous wounds, etc. Examples of sores are bed sores, canker sores, chrome sores, cold sores, pressure sores etc. Examples of ulcers are, e.g., peptic ulcer, duodenal ulcer, gastric ulcer, gouty ulcer, diabetic ulcer, hypertensive ischemic ulcer, stasis ulcer, ulcus cruris (venous ulcer), sublingual ulcer, submucous ulcer, symptomatic ulcer, trophic ulcer, tropical ulcer, veneral ulcer, e.g. caused by gonorrhoea (including urethritis, endocervicitis and proctitis). Conditions related to wounds or sores which may be successfully treated according to the invention are burns, anthrax, tetanus, gas gangrene, scalatina, erysipelas, sycosis barbae, folliculitis, impetigo contagiosa, or impetigo bullosa, etc. There is often a certain overlap between the use of the terms "wound" and "ulcer" and "wound" and "sore" and, furthermore, the terms are often used at random. Therefore as mentioned above, in the present context the term "wounds" encompasses the term "ulcer", "lesion", "sore" and "infarction", and the terms are indiscriminately used unless otherwise indicated.

The kinds of wounds to be treated according to the invention include also i) general wounds such as, e.g., surgical, traumatic, infectious, ischemic, thermal, chemical and bullous wounds; ii) wounds specific for the oral cavity such as, e.g., post-extraction wounds, endodontic wounds especially in connection with treatment of cysts and abscesses, ulcers and lesions of bacterial, viral or autoimmunological origin, mechanical, chemical, thermal, infectious and lichenoid wounds; herpes ulcers, stomatitis aphthosa, acute necrotising ulcerative gingivitis and burning mouth syndrome are specific examples; and iii) wounds on the skin such as, e.g., neoplasm, burns (e.g. chemical, thermal), lesions (bacterial, viral, autoimmunological), bites and surgical incisions. Another way of classifying wounds is as i) small tissue loss due to surgical incisions, minor abrasions and minor bites, or as ii) significant tissue loss. The latter group includes ischemic ulcers, pressure sores, fistulae, lacerations, severe bites, thermal burns and donor site wounds (in soft and hard tissues) and infarctions.

In other aspects of the invention, the wound to be prevented and/or treated is selected from the group consisting of aseptic wounds, infarctions, contused wounds, incised wounds, lacerated wounds, non-penetrating wounds, open wounds, penetrating wounds, perforating wounds, puncture wounds, septic wounds and subcutaneous wounds.

Other wounds which are of importance in connection with the present invention are wounds like ischemic ulcers, pressure sores, fistulae, severe bites, thermal burns and donor site wounds.

Ischemic ulcers and pressure sores are wounds, which normally only heal very slowly and especially in such cases an improved and more rapid healing is of course of great importance for the patient. Furthermore, the costs involved in the treatment of patients suffering from such wounds are markedly reduced when the healing is improved and takes place more rapidly.

Donor site wounds are wounds which e.g. occur in connection with removal of hard tissue from one part of the body to another part of the body e.g. in connection with transplantation. The wounds resulting from such operations are very painful and an improved healing is therefore most valuable.

The term "skin" is used in a very broad sense embracing the epidermal layer of the skin and-in those cases where the skin surface is more or less injured—also the dermal layer of the skin. Apart from the stratum corneum, the epidermal layer of the skin is the outer (epithelial) layer and the deeper connective tissue layer of the skin is called the dermis.

Since the skin is the most exposed part of the body, it is particularly susceptible to various kinds of injuries such as, e.g., ruptures, cuts, abrasions, burns and frostbites or injuries arising from various diseases. Furthermore, much skin is often destroyed in accidents. However, due to the important barrier and physiologic function of the skin, the integrity of the skin is important to the well-being of the individual, and any breach or rupture represents a threat that must be met by the body in order to protect its continued existence.

Apart from injuries on the skin, injuries may also be present in all kinds of tissues (i.e. soft and hard tissues). Injuries on soft tissues including mucosal membranes and/or skin are especially relevant in connection with the present invention.

Healing of a wound on the skin or on a mucosal membrane undergoes a series of stages that results either in repair or regeneration of the skin or mucosal membrane. In recent years, regeneration and repair have been distinguished as the two types of healing that may occur. Regeneration may be defined as a biological process whereby the architecture and function of lost tissue are completely renewed. Repair, on the other hand, is a biological process whereby continuity of disrupted tissue is restored by new tissues which do not replicate the structure and function of the lost ones.

The majority of wounds heal through repair, meaning that the new tissue formed is structurally and chemically unlike the original tissue (scar tissue). In the early stage of the tissue repair, one process which is almost always involved is the formation of a transient connective tissue in the area of tissue injury. This process starts by formation of a new extracellular collagen matrix by fibroblasts. This new extracellular collagen matrix is then the support for a connective tissue during the final healing process. The final healing is, in most tissues, a scar formation containing connective tissue. In tissues which have regenerative properties, such as, e.g., skin and bone, the final healing includes regeneration of the original tissue. This regenerated tissue has frequently also some scar characteristics, e.g. a thickening of a healed bone fracture.

Under normal circumstances, the body provides mechanisms for healing injured skin or mucosa in order to restore the integrity of the skin barrier or the mucosa. The repair process for even minor ruptures or wounds may take a period of time extending from hours and days to weeks. However, in ulceration, the healing can be very slow and the wound may persist for an extended period of time, i.e. months or even years.

Burns are associated with reduced testosterone levels, and hypgonadism is associated with delayed wound healing. In one embodiment, the methods of this invention, provide for treating a subject suffering from a wound or a burn via the administration of a SARM. In one embodiment, the SARM promotes resolving of the burn or wound, or in another embodiment, participates in the healing process of a burn or a wound, or in another embodiment, treats a secondary complication of a burn or wound, for example, via anabolic activity.

In one embodiment, the treatment of burns or wounds further incorporates the use of additional growth factors like epidermal growth factor (EGF), transforming growth factor-α (TGF-α), platelet derived growth factor (PDGF), fibroblast growth factors (FGFs) including acidic fibroblast growth factor (α-FGF) and basic fibroblast growth factor (β-FGF), transforming growth factor-β (TGF-β) and insulin like growth factors (IGF-1 and IGF-2), or any combination thereof, which are promoters of wound healing.

Wound healing may be measured by many procedures known in the art, including wound tensile strength, hydroxyproline or collagen content, procollagen expression, and re-epithelialization. As an example, a SARM as described herein is administered orally or topically, at a dosage of about 0.1-1 mg per day. Therapeutic effectiveness is measured as effectiveness in enhancing wound healing. Enhanced wound healing may be measured by known techniques such as decrease in healing time, increase in collagen density, increase in hydroxyproline, reduction in complications, increase in tensile strength, and increased cellularity of scar tissue.

In one embodiment, the invention relates to the prevention and treatment of diseases, disorders and/or conditions involving involuntary weight loss and/or hypgonadism, which in turn are treated by the administration of a selective androgen receptor modulator. SARMs have been shown to possess anabolic and androgenic activity, which is tissue-selective in activity. In diseases/disorders/conditions, which are exacerbated due to, or a product of hypogonadism, the SARM selected for treatment, in one embodiment, will have enhanced androgenic activity, in one embodiment, and in another embodiment, diseases/disorders/conditions, which are exacerbated due to, or a product of involuntary weight loss/catabolism/cachexia, the SARM selected for treatment will have marked anabolic activity. In some embodiments, the diseases/disorders/conditions being treated are exacerbated due to, or a product of disturbance of the normal hormonal balance and catabolic effects, and a balance of anabolic and androgenic activity is desired, which in one embodiment, is reflected by the SERM selected for use, or combination thereof.

It is to be understood that in any method as described herein, other agents, some of which are described herein, or as known in the art, which are useful in treating these conditions may be incorporated, as well.

The following examples are presented in order to more fully illustrate certain embodiments of the invention. They should in no way be construed, however, as limiting the broad scope of the invention.

### EXPERIMENTAL DETAILS SECTION

### EXAMPLE 1

### Effect of Selective Androgen Receptor Modulators (SARMS) on Subjects with End-Stage Renal Disease

### Compounds tested:

The following Compounds, which are ligands for the AR with potent binding affinity, exhibiting tissue-selective androgenic and anabolic effects, and oral bioavailability, will be assessed for their effects on end-stage renal disease (ESRD).

Compound V:

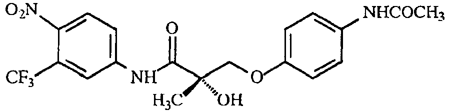

Compound VI:

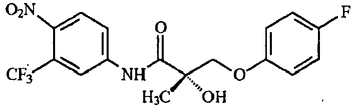

Compound X:

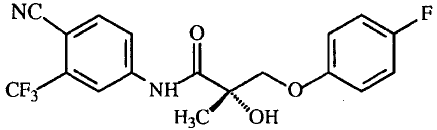

Compound XI:

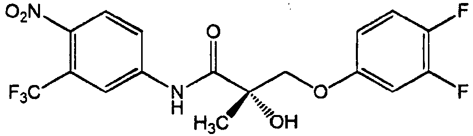

Compound XII:

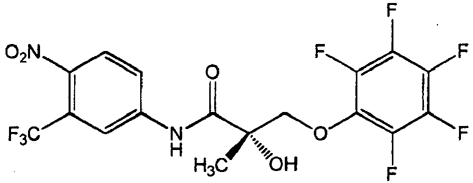

Compound XVI:

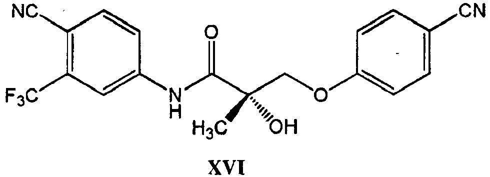

Compound XVII:

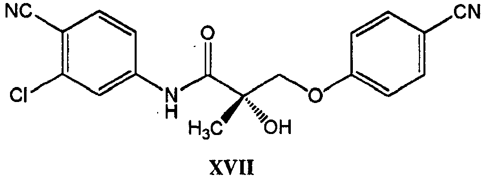

Compound XVIII:

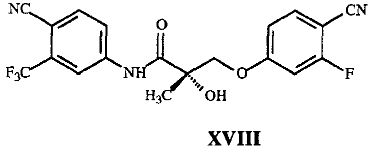

Compound XIX:

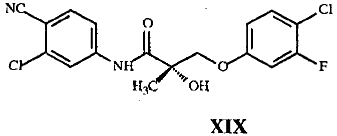

Compound XX:

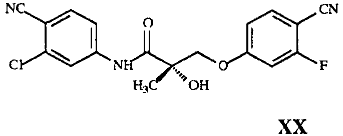

The compounds will be synthesized and characterized, as described for example in United States Patent Application Serial Numbers 10/277,108, 10/270,732 and 10/371,155.

For example, the process for preparing a SARM compound represented by the structure of formula (I): 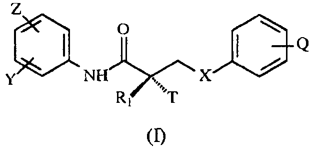 with substituents as described herein, may comprise the step of coupling an amide of formula (XXII): 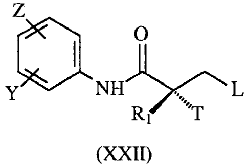 wherein Z, Y, R₁ and T are as defined above and L is a leaving group,
with a compound of formula (XXIII): 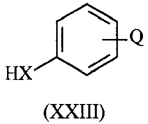 wherein Q and X are as defined above.

In one embodiment, the amide of formula XXII is prepared by the following steps:
a) preparing a carboxylic acid of formula XXV by ring opening of a cyclic compound of formula XXIV 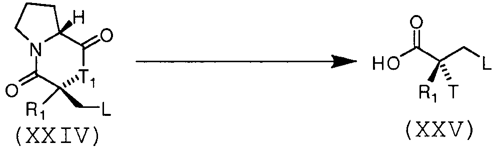 wherein L, R₁ and T are as defined above, and T₁ is O or NH; and
b) reacting an amine of formula XXVI: 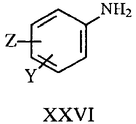 wherein Z and Y are as defined above, with the carboxylic acid of formula XXV in the presence of a coupling reagent, to produce the amide of formula XXII.

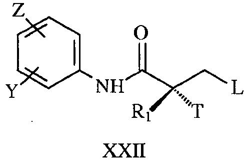

In one embodiment, step (a) is carried out in the presence of HBr.

In one embodiment, whereby compound XXV of step (a) is reacted with a coupling agent prior to step (b).

In another embodiment, the process for preparing a SARM compound: 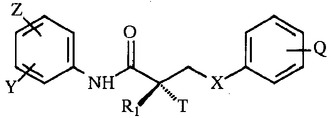 may comprise the steps of:
a) reacting a ring of formula: 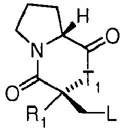 wherein L, R₁ are as defined above, and T₁ is O or NH with a compound of: 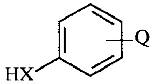 to produce a compound of formula: 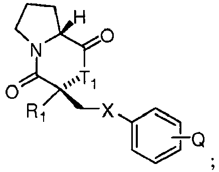
b) ring opening of compound produced in a to produce a compound of formula: 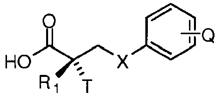 wherein R₁, T, X and Q are as defined above; and
c) coupling the carboxylic acid of compound in (b) with the amine of formula: 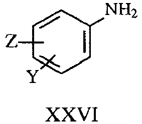 wherein Z and Y are as defined above, in the presence of a coupling reagent, to produce the desired SARM compound.

For example, compound XVI was prepared as follows:

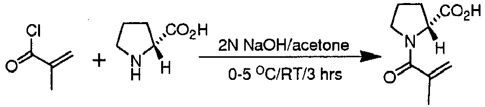

**(2*R*)-1-Methacryloylpyrrolidin-2-carboxylic Acid**. *D*-Proline, 14.93 g, 0.13 mol) was dissolved in 71 mL of 2 N NaOH and cooled in an ice bath; the resulting alkaline solution was diluted with acetone (71 mL). An acetone solution (71 mL) of metacryloly chloride (13.56 g, 0.13 mol) and 2N NaOH solution (71 mL) were simultaneously added over 40 min to the aqueous solution of *D*-proline in an ice bath. The pH of the mixture was kept at 10-11°C during the addition of the metacryloly chloride. After stirring (3 h, room temperature), the mixture was evaporated in vacuo at a temperature at 35-45 °C to remove acetone. The resulting solution was washed with ethyl ether and was acidified to pH 2 with concentrated HCl. The acidic mixture was saturated with NaCI and was extracted with EtOAc (100 mL x 3). The combined extracts were dried over Na₂SO₄, filtered through Celite, and evaporated in vacuo to give the crude product as a colorless oil. Recrystallization of the oil from ethyl ether and hexanes afforded 16.2 (68%) of the desired compound as colorless crystals: mp 102-103 °C (lit. [214] mp 102.5-103.5 °C); the NMR spectrum of this compound demonstrated the existence of two rotamers of the title compound. ¹H NMR (300 MHz, DMSO-d₆) δ 5.28 (s) and 5.15 (s) for the first rotamer, 5.15 (s) and 5.03 (s) for the second rotamer (totally 2H for both rotamers, vinyl CH₂), 4.48-4.44 for the first rotamer, 4.24-4.20 (m) for the second rotamer (totally 1H for both rotamers, CH at the chiral canter), 3.57-3.38 (m, 2H, CH₂), 2.27-2.12 (1H, CH), 1.97-1.72 (m, 6H, CH₂, CH, Me); ¹³C NMR (75 MHz, DMSO-d₆) δ for major rotamer 173.3, 169.1, 140.9, 116.4, 58.3, 48.7, 28.9, 24.7, 19.5: for minor rotamer 174.0, 170.0, 141.6, 115.2, 60.3, 45.9, 31.0, 22.3, 19.7; IR (KBr) 3437 (OH), 1737 (C=O), 1647 (CO, COOH), 1584,1508, 1459, 1369, 1348, 1178 cm⁻¹; [α]_{D}²⁶+80.8° (c=1, MeOH); Anal. Calcd. for C₉H₁₃NO₃: C59.00, H 7.15, N 7.65. Found: C 59.13, H 7.19, N 7.61.

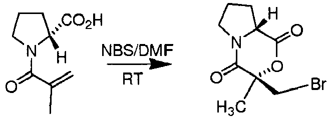

**(3*R*,8a*R*)-3-Bromomethyl-3-methyl-tetrahydro-pyrrolo[2,1-*c*][1,4]oxazine-1,4-dione.** A solution of NBS (23.5g, 0.132 mol) in 100 mL of DMF was added dropwise to a stirred solution of the (methylacryloyl)-pyrrolidine (16.1g, 88 mmol) in 70 mL of DMF under argon at room temperature, and the resulting mixture was stirred 3 days. The solvent was removed in vacuo, and a yellow solid was precipitated. The solid was suspended in water, stirred overnight at room temperature, filtered, and dried to give 18.6 (81 %) (smaller weight when dried - 34%) of the title compound as a yellow solid: mp 152-154 °C (lit. [214] mp 107-109 °C for the S-isomer); ¹H NMR (300 MHz, DMSO-d₆) δ 4.69 (dd, *J* = 9.6 Hz, *J* = 6.7 Hz, 1H, CH at the chiral center), 4.02 (d, *J* = 11.4 Hz, 1H, CHHₐ), 3.86 (d, *J* = 11.4 Hz,1H, CHH_{b}), 3.53-3.24 (m, 4H, CH₂), 2.30-2.20 (m, 1H, CH), 2.04-1.72 (m, 3H, CH₂ and CH), 1.56 (s, 2H, Me); ¹³C NMR (75 MHz, DMSO-d₆) δ 167.3, 163.1, 83.9, 57.2, 45.4, 37.8, 29.0, 22.9, 21.6; IR (KBr) 3474, 1745 (C=O), 1687 (C=O), 1448, 1377, 1360, 1308, 1227, 1159, 1062cm⁻¹; [α]_{D}²⁶ +124.5 °(c =1.3, chloroform); Anal. Calcd. for C₉H₁₂BrNO₃: C 41.24, H 4.61, N 5.34. Found: C 41.46, H 4.64, N 5.32.

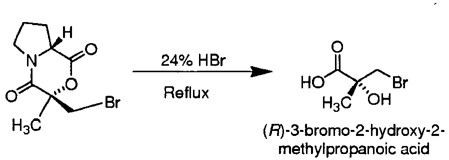

**(2*R*)-3-Bromo-2-hydroxy-2-methylpropanoic Acid.** A mixture of bromolactone (18.5g, 71 mmol) in 300 mL of 24% HBr was heated at reflux for 1 h. The resulting solution was diluted with brine (200 mL), and was extracted with ethyl acetate (100 mL x 4). The combined extracts were washed with saturated NaHCO₃ (100 mL x 4). The aqueous solution was acidified with concentrated HCI to pH =1, which, in turn, was extracted with ethyl acetate (100 mL x 4). The combined organic solution was dried over Na₂SO₄, filtered through Celite, and evaporated in vacuo to dryness. Recrystallization from toluene afforded 10.2 g (86%) of the desired compound as colorless crystals: mp 107-109 °C (lit. [214] mp 109-113 °C for the S-isomer); ¹H NMR (300 MHz, DMSO-d₆) δ 3.63 (d, *J* = 10.1 Hz, 1H, CHHa), 3.52 (d, *J* = 10.1 Hz, 1H, CHH_{b}), 1.35 (s, 3H, Me); IR (KBr) 3434 (OH), 3300-2500 (COOH), 1730 (C=O), 1449, 1421, 1380, 1292, 1193, 1085 cm⁻¹; [α]_{D}²⁶+10.5° (c = 2.6, MeOH); Anal. Calcd. for C₄H₇BrO₃ C 26.25, H 3.86. Found: C 26.28, H 3.75.

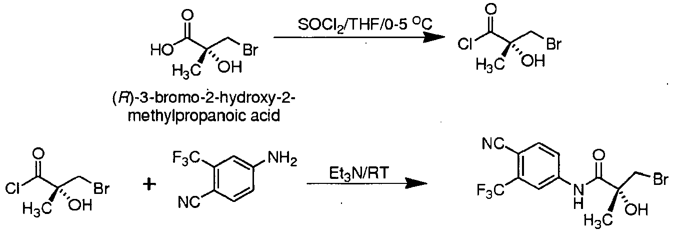

**Synthesis of (2*R*)-3-Bromo-*N*-[4-cyano-3-(trifluoromethyl)phenyl]-2-hydroxy-2-methylpropanamide.** Thionyl chloride (46.02 g, 0.39 mol) was added dropwise to a cooled solution (less than 4 °C) of *R*-131 (51.13 g, 0.28 mol) in 300 mL of THF under an argon atmosphere. The resulting mixture was stirred for 3 h under the same condition. To this was added Et₃N (39.14 g, 0.39 mol) and stirred for 20 min under the same condition. After 20 min, 5-amino- 2-cyanobenzotrifluoride (40.0 g, 0.21 mol), 400 mL of THF were added and then the mixture was allowed to stir overnight at room temperature,The solvent was removed under reduced pressure to give a solid which was treated with 300 mL of H₂O, extracted with EtOAc (2 X 400 mL). The combined organic extracts were washed with saturated NaHCO₃ solution (2 X 300 mL) and brine (300 mL). The organic layer was dried over MgSO₄ and concentrated under reduced pressure to give a solid which was purified from column chromatography using CH₂Cl₂/EtOAc (80:20) to give a solid. This solid was recrystallized from CH₂Cl₂/hexane to give 55.8 g (73.9%) of (2*R*)-3-Bromo-*N*-[4-cyano-3-(trifluoromethyl)phenyl]-2-hydroxy-2-methylpropanamide as a light-yellow solid.

¹H NMR (CDCl₃/TMS) δ 1.66 (s, 3H, C*H₃*), 3.11 (s, 1H, O*H*), 3.63 (d, *J*=10.8 Hz, 1H, C*H₂*),4.05 (d, *J* = 10.8 Hz, 1H, C*H₂*), 7.85 (d, *J* = 8.4 Hz, 1H, Ar*H*), 7.99 (dd, *J* = 2.1, 8.4 Hz, 1H, Ar*H*), 8.12 (d, *J =* 2.1 Hz, 1H, Ar*H*), 9.04 (bs, 1H, N*H*). Calculated Mass: 349.99, [M-H]⁻ 349.0. M.p.: 124-126 °C.

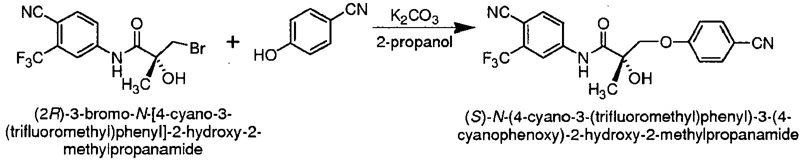

**Synthesis of (*S*)-*N*-(4-Cyano-3-(trifluoromethyl)phenyl)-3-(4-cyanophenoxy)-2-hydroxy-2-methylpropanamide.** A mixture of bromoamide ((2*R*)-3-Bromo-*N*-[4-cyano-3-(trifluoromethyl)phenyl]-2-hydroxy-2-methylpropanamide, 50 g, 0.14 mol), anhydrous K₂CO₃ (59.04 g, 0.43 mol), 4-cyanophenol (25.44 g, 0.21 mol) in 500 mL of 2-propanol was heated to reflux for 3 h and then concentrated under reduced pressure to give a solid. The resulting residue was treated with 500 mL of H₂O and then extracted with EtOAc (2 X 300 mL). The combined EtOAc extracts were washed with 10% NaOH (4 X 200 mL) and brine. The organic layer was dried over MgSO₄ and then concentrated under reduced pressure to give an oil which was treated with 300 mL of ethanol and an activated carbon. The reaction mixture was heated to reflux for 1 h and then the hot mixture was filtered through Celite. The filtrate was concentrated under reduced pressure to give an oil. This oil was purified by column chromatography using CH₂Cl₂/EtOAc (80:20) to give an oil which was crystallized from CH₂Cl₂/hexane to give 33.2 g (59.9%) of (S)-*N*-(4-cyano-3-(trifluoromethyl)phenyl)-3-(4-cyanophenoxy)-2-hydroxy-2-methylpropanamide as a colorless solid (a cotton type).

¹H NMR (CDCl₃/TMS) δ 1.63 (s, 3H, *CH₃),* 3.35 (s, 1H,OH), 4.07 (d, *J* = 9.04 Hz, 1H, C*H*), 4.51 (d, *J* = 9.04 Hz, 1H, CH), 6.97 - 6.99 (m, 2H, ArH), 7.57-7.60 (m, 2H, ArH), 7.81 (d, *J* = 8.55 Hz, 1H, ArH), 7.97 (dd, *J* = 1.95, 8.55 Hz, 1H, Ar*H*), 8.12 (d, *J* = 1.95 Hz, 1H, *A*r*H),* 9.13 (bs, 1H, N*H*). Calculated Mass: 389.10, [M-H]- 388.1. Mp: 92-94 °C.

### ESRD model:

Rat partial nephrectomy and rat remnant kidney model (RRKM) models are employed essentially as described (Vukicevic, et al. (1987) J. Bone Mineral Res. 2:533).

Male rats (2-3 months old, weighing about 150-200 g) are subjected to unilateral nephrectomy (either left or right kidney). After approximately one week, 2/3 of the remaining kidney is surgically removed. Immediately following surgery, plasma creatinine and BUN levels rise dramatically due to the loss of renal mass and function. Over the next several weeks of this "acute" failure phase, plasma creatinine and BUN levels of surviving animals decline somewhat toward normal values but remain elevated. Renal function then appears to remain relatively constant or stable for a period of variable duration. After this point, the animals enter a period of chronic renal failure in which there is an essentially linear decline in renal function ending in death.

As surgical controls, additional rats are subjected to a "sham" operation in which the kidneys are decapsulated but no renal tissue is removed.

### Effect of treatment on ESRD

Nephrectomized and sham-operated rats are maintained for approximately 5-6 months after surgery, a point at which the animals have entered chronic renal failure. Rats are then divided into groups including controls, which receive no treatment or placebo, those receiving the compounds and those receiving the respective compounds and erythropoietin.

All drugs are given to animals as freshly prepared solutions in polyethylene glycol 300 (PEG 300). Compounds are delivered in osmotic pumps, which are implanted subdurally in the animals. After 14 days of treatment, rats are weighed, anesthetized, and sacrificed. Osmotic pumps are also removed from animals to check for correct pump operation

Mortality is assessed, as well as serum creatinine and/or urea levels, with therapeutic effects correlating with reduction of mortality and/or serum levels, as compared to controls.

Kidney tissue is processed for histological observation, in order to determine effects of treatment glomerular histology. In particular, incidence or reduction thereof of glomerular sclerosis and loop collapse, scattered sclerosis and microaneurysms, are assessed.

Another model for chronic renal failure is using rats subjected to partial nephrectomies, and allowed to recover for approximately two weeks after surgery before initiation of therapy. At this point, surviving animals are past the acute renal failure phase and have not yet entered chronic renal failure.

Rats are similarly divided into groups and treated as described above. Serum and urine creatinine levels are similarly assessed, as are tissue samples for assessement of the preservation or maintenance of glomeruli, and proximal/distal tubule structures.

### EXAMPLE 2

### Treatment of Subjects with Skin Ulcerations or Burns with SARMs

In order to determine whether the compounds of this invention are useful in treating skin wounds and/or burns, representative compounds, such as those in Example 1 are prepared as a topical formulation, and in another embodiment, an intravenous formulation. Patients presenting with cutaneous ulcers or burns are assessed.

In subjects with wounds, the wounds may be debrided and the SARM applied topically, or standard therapy including antiseptics and chemotherapeutics may be administered to the wound site, and the SARM is provided orally or intravenously.

In subjects with cutaneous ulcers or bums, the SARMs may be applied topically, or intravenously, as described, alone, or in combination with other known therapies, such as antibiotics, growth factors, etc.

The SARM treatment may be repeated over time, and gross evaluation of the wound or burn site is evaluated, pain is assessed.

Other parameters evaluated may include determination of the percent body weight loss, and loss of muscle mass. Another group of subjects may comprise those given a high calorie, high protein diets which may include vitamin and mineral supplements.

Standard animal models of burns and/or wounds may also be evaluated in this context. For example, Sprague-Dawley rats given a standard contact burn (20% TBSA), are evaluated. On day 3, wounds are excised and infected with *Pseudomonas aeruginosa* and *Staphylococcus aureus* at 5.0 x 10⁵ cfu/ml. The animals are then divided into treatment groups, and treated as described.

### EXAMPLE 3

### Treatment of Subjects with Spinal Cord Injuries with SARMs

Animal models of spinal cord injuries are evaluated as follows: fully adult (approximately 400 grams (g)) female guinea pigs (Hartley Strain) are anesthetized with ketamine/xylyzine by conventional methods (Borgens et al. (2002) J. Exp. Biol. 205,1-12) prior to surgery, kept warm with heat lamps after surgery, and maintained individually in pens and fed ad libidum. Animals were euthanized at the end of the study, prior to the harvesting of spinal cords for anatomical study by an overdose of the anesthesia (see Borgens et al. (2002) J. Exp. Biol. 205, 1-12).

A laminectomy procedure exposing the dorsal aspect of the spinal cord was performed on all animals between T9 and T11. A right lateral hemisection is performed, and confirmed to be "complete" (leaving no spared parenchyma) by passing a sharpened pin through the cut tissue. This operation severs the entire right side of the cord from the midline to the far right border of the spinal cord forming a rostral and caudal segment. The entire left side of the spinal cord is left intact. Immediately after transection, a marker device made of surgical stainless steel is inserted into the lesion, as previously described in Borgens et al. (1986) J. Comp. Neurol 250,168-180 and Borgens and Bohnert (1997) Exp. Neurol. 145,376-389, which is left *in situ* for the duration of the study, and removed prior to histological processing. This procedure leaves a hole in the tissue, which accurately marks the exact plane of transaction even in chronic injuries many months old.

In addition, subjects with spinal cord injury (SCI) may be treated experimentally with SARMs. Human studies have shown that central neurotransmitters may be altered after SCI and thus cause a hypothalamus-pituitary-adrenal axis dysfunction, leading to a decrease in testosterone and other hormone levels.

Moreover, the effect of SCI or other acute illness or trauma characteristically includes heightened catabolism in conjunction with the lowered anabolic activity resulting in a condition that is prone to loss of lean body tissue. As long as the catabolic process goes uninterrupted, disturbed nutrient utilization will continue. The effects of the loss of lean body mass include the development of wounds and impaired healing mechanisms. Because of poor nutrition and protein combined with immobilization, patients with spinal cord injury are at high risk for bed sores.

As described in Example 2, SARMs are useful in treating wounds, enhancing muscle mass, and diminishing cachexia, thus SARM treatment in SCI subjects will be evaluated, and its effect on these conditions assessed.

It will be appreciated by a person skilled in the art that the present invention is not limited by what has been particularly shown and described hereinabove. Rather, the scope of the invention is defined by the claims which follow:

## Claims

1. A selective androgen receptor modulator (SARM) compound having the formula I: 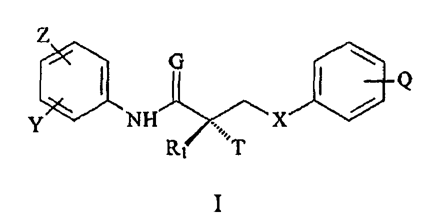
wherein G is O or S;
X is O;
T is OH, OR, NHCOCH₃, or NHCOR;
Z is NO₂, CN, COOH, COR, NHCOR or CONHR;
Y is CF₃, F, I, Br, Cl, CN, C(R)₃ or Sn(R)₃;
Q is alkyl, halogen, CF₃, CN, C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR, NHSO₂CH₃, NHSO₂R, OR, COR, OCOR, OSO₂R, SO₂R, SR; or Q together with the benzene ring to which it is attached is a fused ring system represented by structure A, B or C: 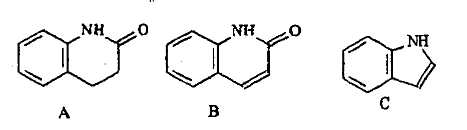
R is alkyl, haloalkyl, dihaloalkyl, trihaloalkyl, CH₂F, CHF₂, CF₃, CF₂CF₃, aryl, phenyl, halogen, alkenyl or OH; and
R₁ is CH₃, CH₂F, CHF₂, CF₃, CH₂CH₃, or CF₂CF₃
for use in treating, preventing, suppressing, inhibiting or reducing the incidence of symptoms associated with kidney disease, wherein said symptoms associated with kidney disease are hypogonadism in kidney disease, or wherein said kidney disease is end-stage renal disease (ESRD).

2. A SARM compound for use according to claim 1, wherein said symptoms associated with end-stage renal disease (ESRD) are selected from hypogonadism, involuntary weight loss and fatigue.

3. A SARM compound for use according to claim 1 or 2, wherein said SARM compound is represented by the structure of formula II: 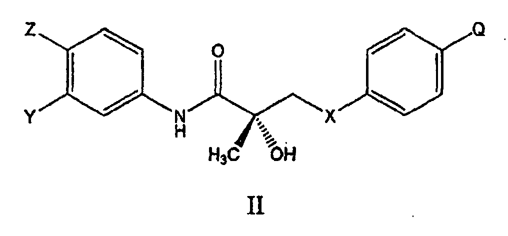
wherein X is O;
Z is NO₂, CN, COOH, COR, NHCOR or CONHR;
Y is CF₃, F, I, Br, Cl, CN, C(R)₃ or Sn(R)₃;
Q is alkyl, halogen, CF₃, CN, C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR NHS0₂CH₃, NHSO₂R, OR, COR, OCOR, OSO₂R SO₂R, SR; or Q together with the benzene ring to which it is attached is a fused ring system represented by structure A, B or C: 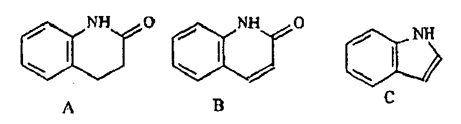
R is alkyl, haloalkyl, dihaloalkyl, trihaloalkyl, CH₂F, CHF₂, CF₃, CF₂CF₃, aryl, phenyl, halogen, alkenyl or OH.

4. A SARM compound for use according to claim 3, wherein Y is CF₃.

5. A SARM compound for use according to claim 3, wherein Z is NO₂.

6. A SARM compound for use according to claim 3, wherein Z is CN.

7. A SARM compound for use according to claim 3, wherein Q is halogen.

8. A SARM compound for use according to claim 3, wherein Z is CN, Y is CF₃ and Q is F.

9. A SARM compound for use according to claim 3, wherein Z is CN, Y is CF₃ and Q is CN.

10. A SARM compound for use according to claim 3, wherein Z is CN, Y is Cl and Q is CN.

11. A selective androgen receptor modulator (SARM) compound represented by the structure of : 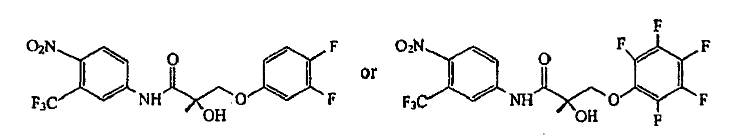 for use in treating, preventing, suppressing, inhibiting or reducing the incidence of symptoms associated with kidney disease, wherein said symptoms associated with kidney disease are hypogonadism in kidney disease.

12. A SARM compound for use according to any of claims 1 to 3, wherein the compound is to be administered as a pharmaceutical composition comprising said SARM and a pharmaceutically acceptable carrier.

13. A SARM compound for use according to claim 12, wherein said compound is to be administered intravenously, intraarterially, or intramuscularly in liquid form; subcutaneously implanted in the form of a pellet containing said pharmaceutical composition; orally administered in a liquid or solid form; or topically applied to the skin surface.

14. A SARM compound for use according to claim 12, wherein said pharmaceutical composition is a pellet, a tablet, a capsule, a solution, a suspension, an emulsion, an elixir, a gel, a cream, a suppository or a parenteral formulation.

15. A SARM compound for use according to claim 3, wherein Z is CN, Y is CF₃ and Q is Cl.

16. A SARM compound for use according to claim 12, wherein said compound is an isomer, pharmaceutically acceptable salt, hydrate, *N*-oxide, or any combination thereof.

## Patentansprüche

1. Eine selektive Androgenrezeptormodulator-(SARM)-Verbindung mit der folgenden Formel I: 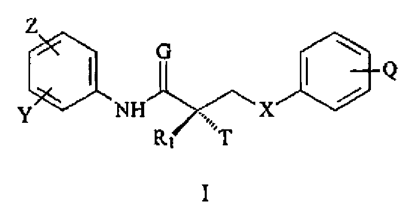
wobei G O oder S ist;
X O ist;
T OH, OR, NHCOCH₃ oder NHCOR ist;
Z NO₂, CN, COOH, COR, NHCOR oder CONHR ist;
Y CF₃, F, I, Br, Cl, CN, C(R)₃ oder Sn(R)₃ ist;
Q Alkyl, Halogen, CF₃, CN, C(R)₃, Sn(R)₃, N(R)₃, NHCOCH₃,NHCOCF₃, NHCOR, NHCONHR, NHCOOR, OCONHR, CONHR, NHCSCH₃, NHCSCF₃, NHCSR, NHSO₂CH₃, NHSO₂R, OR, COR,OCOR,OSO₂R, SO₂R, SR ist; oder Q zusammen mit dem Benzenring, mit dem es verbunden ist, ein kondensiertes Ringsystem ist, das durch die Strukturformel A, B oder C repräsentiert wird: 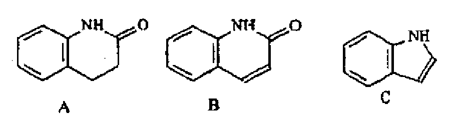
R Alkyl; Haloalkyl, Dihaloalkyl, Trihaloalkyl, CH₂F, CHF₂, CF₃, CF₂CF₃, Aryl, Phenyl, Halogen, Alkenyl oder OH ist; und
R₁ CH₃, CH₂F, CHF₂, CF₃, CH₂CH₃ oder CF₂CF₃ ist;
zur Verwendung in der Behandlung von, Vorbeugung gegen, Unterdrückung von, Blockierung von oder Reduktion von Symptomen, die in Verbindung mit Nierenerkrankung auftreten, wobei die genannten, mit Nierenerkrankung verbundenen Symptome Hypogonadismus bei Nierenerkrankung sind oder wobei die genannte Nierenerkrankung Niereninsuffizienz im Endstadium (ESRD) ist.

2. Eine SARM-Verbindung zur Verwendung entsprechend Anspruch 1, wobei die genannten Symptome, die mit Niereninsuffizienz im Endstadium (ESRD) verbunden sind, von Hypogonadismus, unfreiwilliger Gewichtsabnahme und Ermüdung ausgewählt werden.

3. Eine SARM-Verbindung zur Verwendung entsprechend Anspruch 1 oder 2, wobei die genannte SARM-Verbindung durch die folgende Strukturformel II repräsentiert wird: 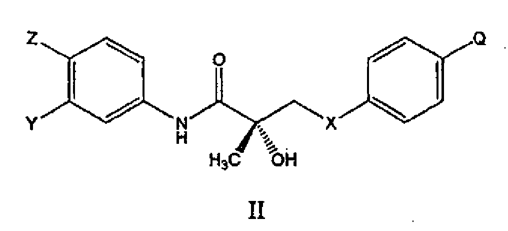
wobei X O ist;
Z NO₂, CN, COOH, COR, NHCOR oder CONHR ist;
Y CF₃, F, I, Br, Cl, CN, C(R)₃ oder Sn(R)₃ ist;
Q Alkyl, Halogen, CF₃, CN, C(R)₃, Sn(R)₃, N(R)₃, NHCOCH₃,NHCOCF₃, NHCOR, NHCONHR, NHCOOR, OCONHR, CONHR, NHCSCH₃, NHCSCF₃, NHCSR, NHSO₂CH₃, NHSO₂R, OR, COR,OCOR,OSO₂R, SO₂R, SR ist; oder Q zusammen mit dem Benzenring, mit dem es verbunden ist, ein kondensiertes Ringsystem ist, das durch die Strukturformel A, B oder C repräsentiert wird: 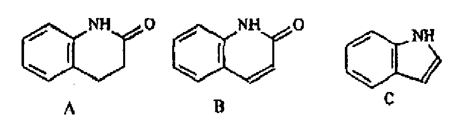
R Alkyl; Haloalkyl, Dihaloalkyl, Trihaloalkyl, CH₂F, CHF₂, CF₃, CF₂CF₃, Aryl, Phenyl, Halogen, Alkenyl oder OH ist.

4. Eine SARM-Verbindung zur Verwendung entsprechend Anspruch 3, wobei Y CF₃ ist.

5. Eine SARM-Verbindung zur Verwendung entsprechend Anspruch 3, wobei Z NO₂ ist.

6. Eine SARM-Verbindung zur Verwendung entsprechend Anspruch 3, wobei Z CN ist.

7. Eine SARM-Verbindung zur Verwendung entsprechend Anspruch 3, wobei Q Halogen ist.

8. Eine SARM-Verbindung zur Verwendung entsprechend Anspruch 3, wobei Z CN, Y CF₃ und Q F ist.

9. Eine SARM-Verbindung zur Verwendung entsprechend Anspruch 3, wobei Z CN, Y CF₃ und Q CN ist.

10. Eine SARM-Verbindung zur Verwendung entsprechend Anspruch 3, wobei Z CN, Y Cl und Q CN ist.

11. Eine selektive Androgenrezeptormodulator-(SARM)-Verbindung, die durch die folgende Strukturformel repräsentiert wird: 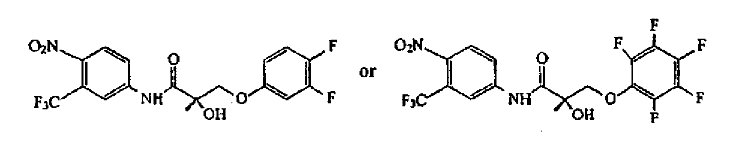 zur Verwendung in der Behandlung von, Vorbeugung gegen, Unterdrückung von, Blockierung von oder Reduktion von Symptomen, die in Verbindung mit Nierenerkrankung auftreten, wobei die genannten, mit Nierenerkrankung verbundenen Symptome Hypogonadismus bei Nierenerkrankung sind.

12. Eine SARM-Verbindung zur Verwendung entsprechend einem der Ansprüche 1 bis 3, wobei die Verbindung als eine pharmazeutische Zusammensetzung verabreicht wird, die sich aus dem genannten SARM und einem pharmazeutisch verträglichen Trägerstoff zusammensetzt.

13. Eine SARM-Verbindung zur Verwendung entsprechend Anspruch 12, wobei die genannte Verbindung intravenös, intraarteriell oder intramuskulär in flüssiger Form, subkutan implantiert in Form eines Pellets, das die genannte pharmazeutische Zusammensetzung enthält, oral in flüssiger oder fester Form verabreicht wird oder topikal auf der Hautoberfläche angewandt wird.

14. Eine SARM-Verbindung zur Verwendung entsprechend Anspruch 12, wobei die genannte Verbindung ein Pellet, eine Tablette, eine Kapsel, ein Lösung, eine Suspension, eine Emulsion, ein Elixier, ein Gel, eine Creme, ein Zäpfchen oder eine parenterale Formulierung ist.

15. Eine SARM-Verbindung zur Verwendung entsprechend Anspruch 3, wobei Z CN, Y CF₃ und Q Cl ist.

16. Eine SARM-Verbindung zur Verwendung entsprechend Anspruch 12, wobei die genannte Verbindung ein Isomer, ein pharmazeutisch verträgliches Salz, ein Hydrat, N-Oxid, oder eine beliebige Kombination dieser ist.

## Revendications

1. Un composé modulateur sélectif du récepteur aux androgènes (SARM) possédant la formule I: 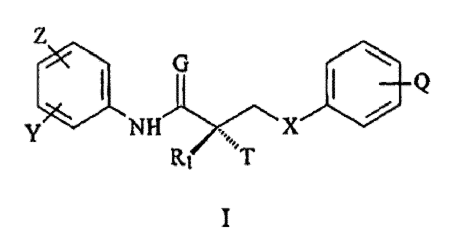
où G est O ou S,
X est O,
T est OH, OR, NHCOCH₃ ou NHCOR,
Z est NO₂, CN, COOH, COR, NHCOR ou CONHR,
Y est CF₃, F, I, Br, CI, CN, C(R)₃ ou Sn(R)₃,
Q est alkyle, halogène, CF₃, CN, C(R)₃, Sn(R)₃, N(R)₂, NHCOCH₃, NHCOCF₃, NHCOR, NHCONHR, NHCOOR, OCONHR, CONHR, NHCSCH₃, NHCSCF₃, NHCSR, NHSO₂CH₃, NHSO₂R, OR, COR, OCOR, OSO₂R, SO₂R, SR, ou Q conjointement avec le cycle benzénique auquel il est attaché est un système de cycle fusionné représenté par la structure A, B ou C : 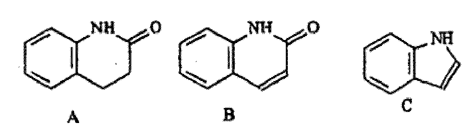
R est alkyle, haloalkyle, dihaloalkyle, trihaloalkyle, CH₂F, CHF₂, CF₃, CF₂CF₃, aryle, phényle, halogène, alcényle ou OH, et
R₁ est CH₃, CH₂F, CHF₂, CF₃, CH₂CH₃, ou CF₂CF₃
pour une utilisation dans le traitement, la prévention, la suppression, l'inhibition ou la réduction de l'incidence de symptômes associés à une maladie rénale, où lesdits symptômes associés à une maladie rénale sont hypogonadisme dans une maladie rénale, ou où ladite maladie rénale est une insuffisance rénale terminale (ESRD).

2. Un composé SARM pour une utilisation selon la Revendication 1, où lesdits symptômes associés à une insuffisance rénale terminale (ESRD) sont sélectionnés parmi hypogonadisme, perte de poids involontaire et fatigue.

3. Un composé SARM pour une utilisation selon la Revendication 1 ou 2, où ledit composé SARM est représenté par la structure de formule II : 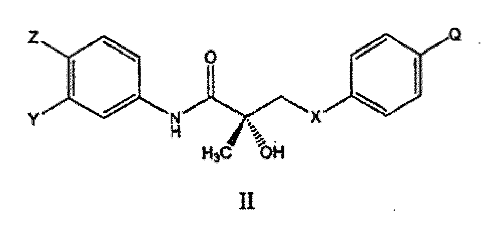
où X est O,
Z est NO₂, CN, COOH, COR, NHCOR ou CONHR,
Y est CF₃, F, I, Br, CI, CN, C(R)₃ ou Sn(R)₃,
Q est alkyle, halogène, CF₃, CN, C(R)₃" Sn(R)₃, N(R)₂, NHCOCH₃, NHCOCF₃, NHCOR, NHCONHR, NHCOOR, OCONHR, CONHR, NHCSCH₃, NHCSCF₃, NHCSR NHSO₂CH₃, NHSO₂R, OR, COR, OCOR, OSO₂R, SO₂R, SR, ou Q conjointement avec le cycle benzénique auquel il est attaché est un système de cycle fusionné représenté par la structure A, B ou C : 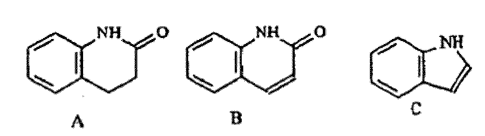
R est alkyle, haloalkyle, dihaloalkyle, trihaloalkyle, CH₂F, CHF₂, CF₃, CF₂CF₃, aryle, phényle, halogène, alcényle ou OH.

4. Un composé SARM pour une utilisation selon la Revendication 3, où Y est CF₃.

5. Un composé SARM pour une utilisation selon la Revendication 3, où Z est NO₂.

6. Un composé SARM pour une utilisation selon la Revendication 3, où Z est CN.

7. Un composé SARM pour une utilisation selon la Revendication 3, où Q est halogène.

8. Un composé SARM pour une utilisation selon la Revendication 3, où Z est CN, Y est CF₃ et Q est F.

9. Un composé SARM pour une utilisation selon la Revendication 3, où Z est CN, Y est CF₃ et Q est CN.

10. Un composé SARM pour une utilisation selon la Revendication 3, où Z est CN, Y est CI et Q est CN.

11. Un composé modulateur sélectif du récepteur aux androgènes (SARM) représenté par la structure de : 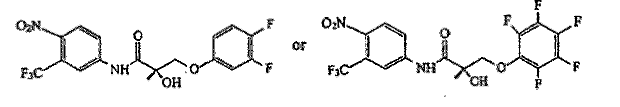 pour une utilisation dans le traitement, la prévention, la suppression, l'inhibition ou la réduction de l'incidence de symptômes associés à une maladie rénale, où lesdits symptômes associés à une maladie rénale sont hypogonadisme dans une maladie rénale.

12. Un composé SARM pour une utilisation selon l'une quelconque des Revendications 1 à 3, où le composé est destiné à être administré sous la forme d'une composition pharmaceutique contenant ledit composé SARM et un excipient pharmaceutiquement acceptable.

13. Un composé SARM pour une utilisation selon la Revendication 12, où ledit composé est destiné à être administré de manière intraveineuse, intraartérielle ou intramusculaire sous forme liquide, de manière sous-cutanée implanté sous la forme d'une pastille contenant ladite composition pharmaceutique, administré oralement sous une forme liquide ou solide, ou appliqué topiquement à la surface de la peau.

14. Un composé SARM pour une utilisation selon la Revendication 12, où ladite composition pharmaceutique est une pastille, un comprimé, une capsule, une solution, une suspension, une émulsion, un élixir, un gel, une crème, un suppositoire ou une formulation parentérale.

15. Un composé SARM pour une utilisation selon la Revendication 3, où Z est CN, Y est CF₃ et Q est Cl.

16. Un composé SARM pour une utilisation selon la Revendication 12, où ledit composé est un isomère, un sel pharmaceutiquement acceptable, un hydrate, un N-oxyde, ou toute combinaison de ceux-ci.
